(19) Europäisches Patentamt
European Patent Office
Office européen des brevets

(11) Publication number: **0 426 021 A1**

(12) **EUROPEAN PATENT APPLICATION**

(21) Application number: **90120543.5**

(22) Date of filing: **26.10.90**

(51) Int. Cl.[5]: **C07D 471/04**, A61K 31/44, C07D 487/04, C07D 473/00, C07D 473/40, C07D 403/10, A61K 31/52, A61K 31/505, A61K 31/415, A61K 31/495, A61K 31/50

(30) Priority: **31.10.89 GB 8924458**
**22.12.89 GB 8929069**
**21.05.90 GB 9011336**

(43) Date of publication of application:
**08.05.91 Bulletin 91/19**

(84) Designated Contracting States:
**AT BE CH DE DK ES FR GB GR IT LI LU NL SE**

(71) Applicant: **FUJISAWA PHARMACEUTICAL CO., LTD.**
**4-7, Doshomachi 3-chome Chuo-ku**
**Osaka-shi Osaka 541(JP)**

(72) Inventor: **Oku, Teruo**
**8-2, Ooazamidorigaoka**
**Tsukuba-shi, Ibaraki 305(JP)**
Inventor: **Setoi, Hiroyuki**
**13-1, Namiki 4-chome**
**Tsukuba-shi, Ibaraki 305(JP)**
Inventor: **Kayakiri, Hiroshi**
**2-5-4-506, Umezono**
**Tsukuba-shi, Ibaraki 305(JP)**
Inventor: **Inoue, Takayuki**
**2-25-10, Matsushiro**
**Tsukuba-shi, Ibaraki 305(JP)**
Inventor: **Kuroda, Akio**
**24-1, Takezono 3-chome**
**Tsukuba-shi, Ibaraki 305(JP)**
Inventor: **Katayama, Akira**
**2-28-38, Umezono**
**Tsukuba-shi, Ibaraki 305(JP)**
Inventor: **Hashimoto, Masashi**
**1-6-17, Nakayamasatsukidai**
**Takarazuka-shi, Hyogo 665(JP)**
Inventor: **Satoh, Sigeki**
**2-3-17 B-101, Higashi**
**Tsukuba-shi, Ibaraki 305(JP)**
Inventor: **Tanaka, Hirokazu**
**1-4-8, Ottominami**
**Tsuchiura-shi, Ibaraki 300(JP)**

(74) Representative: **Türk, Dietmar, Dr. rer. nat. et al**
**Türk, Gille + Hrabal Patentanwälte**
**Brucknerstrasse 20**
**W-4000 Düsseldorf 13(DE)**

(54) **Condensed imidazole derivatives and processes for preparation thereof.**

(57) A compound of the formula :

EP 0 426 021 A1

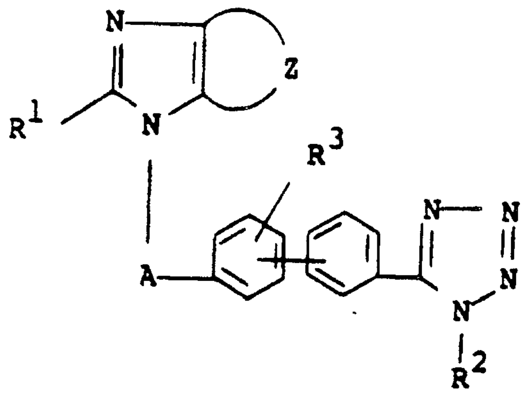

wherein $R^1$ is lower alkyl, halo(lower)alkyl, cyclo(lower)alkyl or lower alkylthio,
$R^2$ is hydrogen or imino-protective group,
$R^3$ is hydrogen or halogen,
A is lower alkylene, and

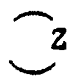

is a group of the formula :

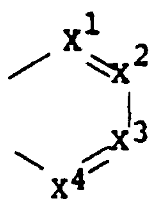 , 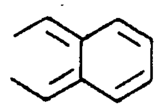 or 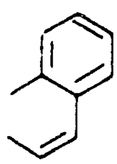

in which
$X^1$, $X^2$, $X^3$ and $X^4$ are each
N or $CR^4$, in which $R^4$ is hydrogen, lower alkyl, lower alkoxy, halogen, halo(lower)alkyl, esterified carboxy, halo-(lower)alkoxy or hydroxy,
provided that at least one of $X^1$, $X^2$, $X^3$ and $X^4$ is N,
and pharmaceutically acceptable salts thereof, processes for their preparation and pharmaceutical compositions comprising them as an active ingredient in admixture with a pharmaceutically acceptable carrier or excipient.

# CONDENSED IMIDAZOLE DERIVATIVES AND PROCESSES FOR PREPARATION THEREOF

The present invention relates to novel condensed imidazole derivatives and a pharmaceutically acceptable salt thereof. More particularly, it relates to novel condensed imidazole derivatives and a pharmaceutically acceptable salt thereof which have pharmacological activities such as angiotensin II antagonism and the like, to processes for preparation thereof, to a pharmaceutical composition comprising the same and to a use of the same as a medicament.

Accordingly, one object of the present invention is to provide novel condensed imidazole derivatives and a pharmaceutically acceptable salt thereof, which are useful as a potent and selective antagonist of angiotensin II receptor.

Another object of the present invention is to provide processes for preparation of said condensed imidazole derivatives or a salt thereof.

A further object of the present invention is to provide a pharmaceutical composition comprising, as an active ingredient, said condensed imidazole derivatives or a pharmaceutically acceptable salt thereof.

Still further object of the present invention is to provide a use of said condensed imidazole derivatives or a pharmaceutically acceptable salt thereof as a medicament such as angiotensin II antagonist useful for treating or preventing angiotensin II mediated diseases, for example, hypertension (e.g. essential hypertension, renal hypertension, etc.), heart failure, and the like in human being or animals.

The condensed imidazole derivatives of the present invention are novel and can be represented by the formula (I) :

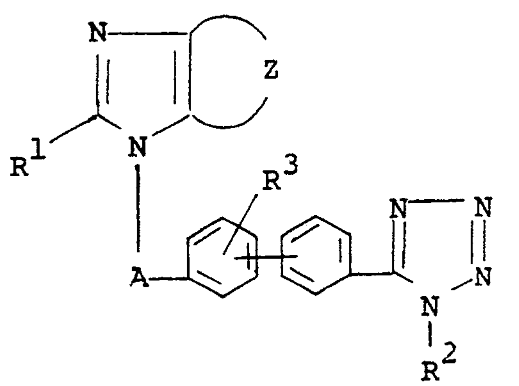

(I)

wherein $R^1$ is lower alkyl, halo(lower)alkyl, cyclo(lower)alkyl or lower alkylthio,
$R^2$ is hydrogen or imino-protective group,
$R^3$ is hydrogen or halogen,
A is lower alkylene, and

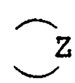

is a group of the formula :

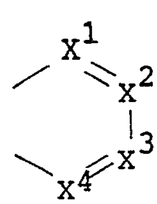 , 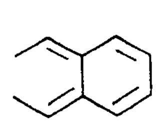 or 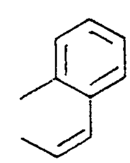

in which

$X^1$, $X^2$, $X^3$ and $X^4$ are each
N or $CR^4$, in which $R^4$ is hydrogen, lower alkyl, lower alkoxyl, halogen, halo(lower)alkyl, esterified carboxy, halo(lower)alkoxy or hydroxy,
provided that at least one of $X^1$, $X^2$, $X^3$ and $X^4$ is N.

According to the present invention, the object compound (I) can be prepared by the following processes.

Process 1

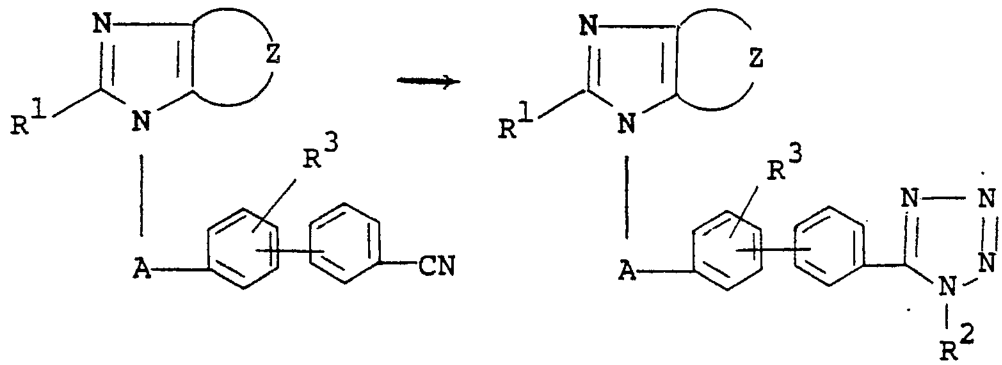

(II)

(I)
or a salt thereof

Process 2

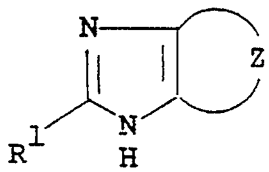

+

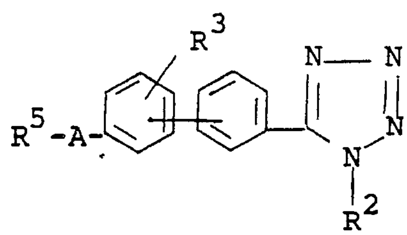

(III)
or a salt thereof

(IV)
or a salt thereof

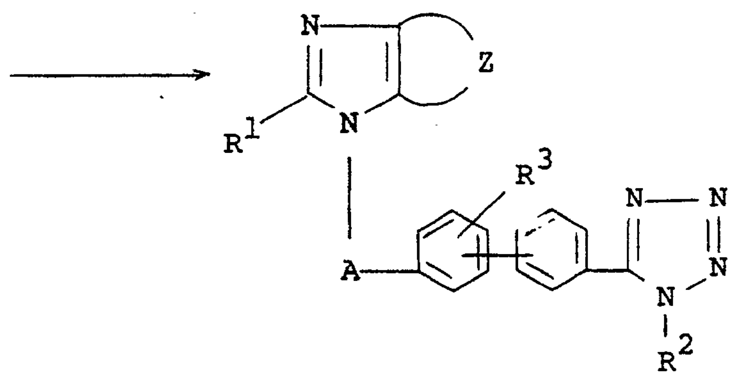

(I)

or a salt thereof

Process 3

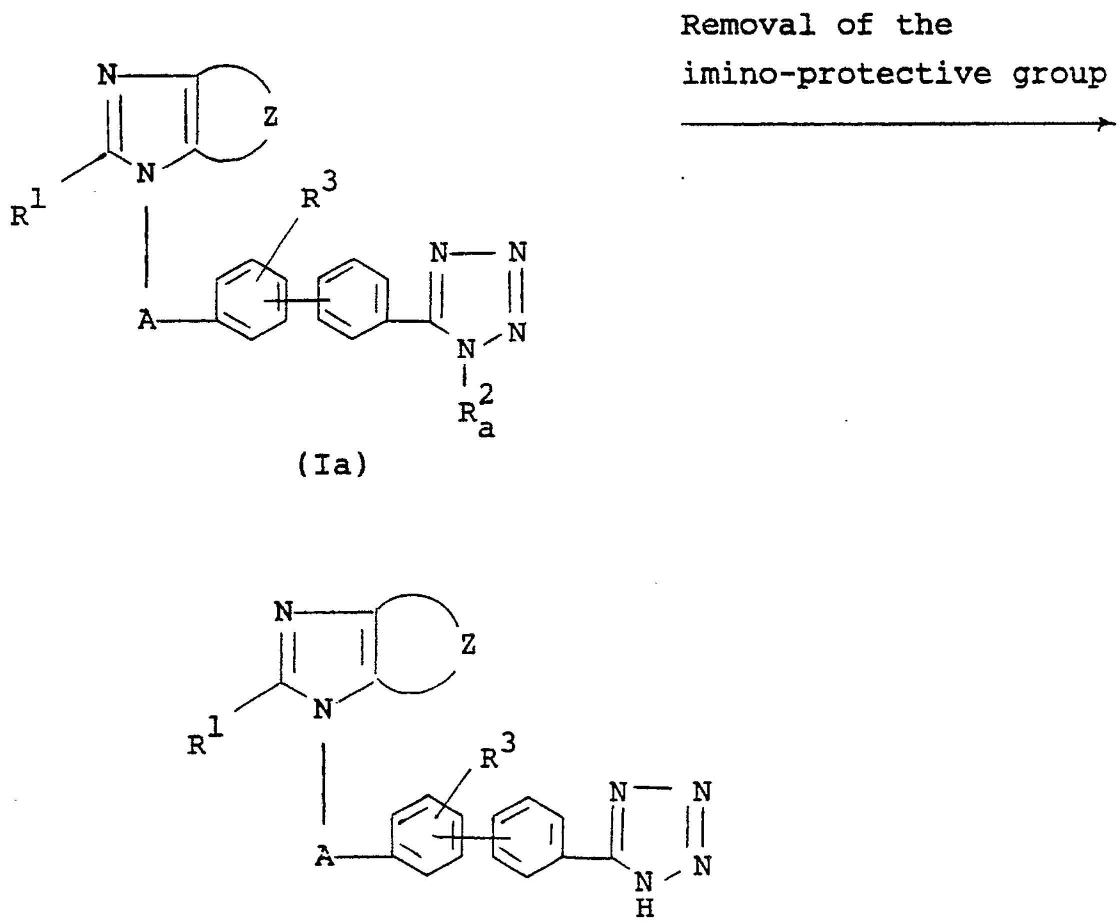


(Ia)

(Ib)

or a salt thereof

Process 4

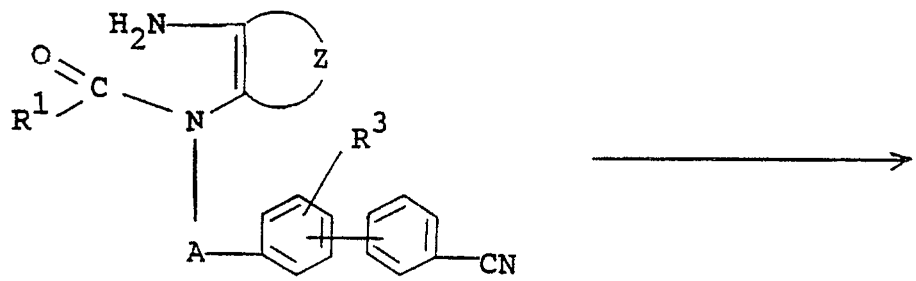

(V)

or a salt thereof

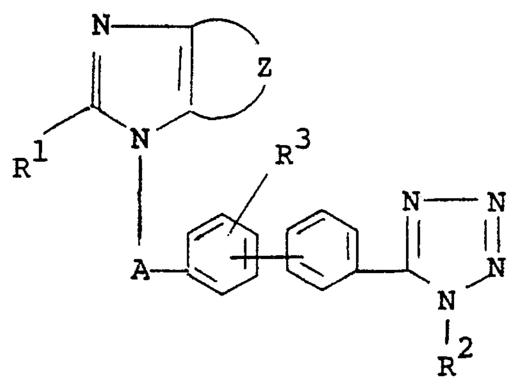

(I)

or a salt thereof

Process 5

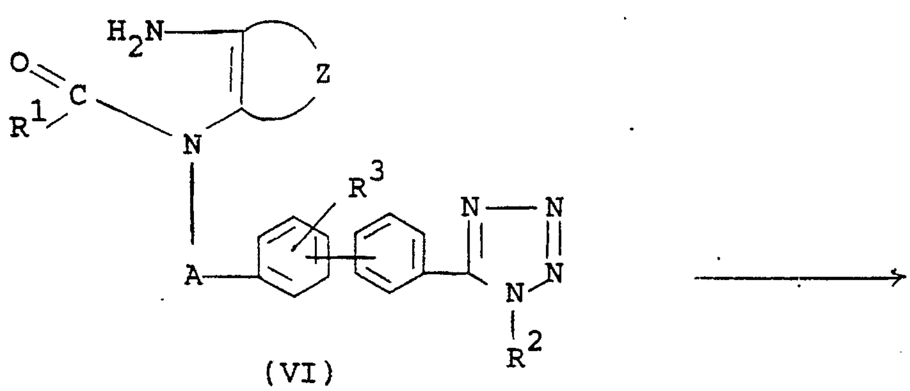

(VI)

or a salt thereof

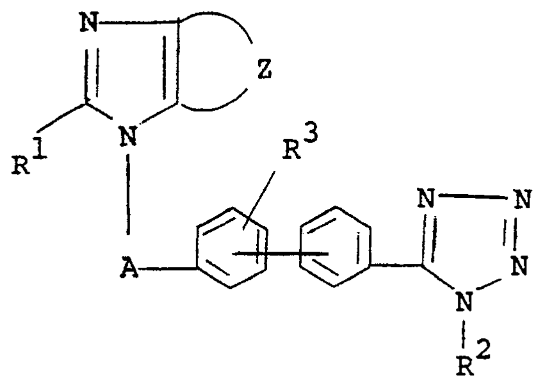

(I)

or a salt thereof

wherein $R^1$, $R^2$, $R^3$, A and

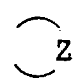

are each as defined above,
$R^2_a$ is imino-protective group, and
$R^5$ is acid residue.

Suitable salts of the compounds (I), (Ib), (III), (IV), (V) and (VI) are conventional non-toxic, pharmaceutically acceptable salt and may include a salt with a base or an acid addition salt such as a salt with an inorganic base, for example, an alkali metal salt (e.g. sodium salt, potassium salt, cesium salt, etc.), an alkaline earth metal salt (e.g. calcium salt, magnesium salt, etc.), an ammonium salt; a salt with an organic base, for example, an organic amine salt (e.g. triethylamine salt, pyridine salt, picoline salt, ethanolamine salt, triethanolamine salt, dicyclohexylamine salt, N,N′-dibenzylethylenediamine salt, etc.), etc.; an inorganic acid addition salt (e.g. hydrochloride, hydrobromide, sulfate, phosphate, etc.); an organic carboxylic or sulfonic acid addition salt (e.g. formate, acetate, trifluoroacetate, maleate, tartrate, methanesulfonate, benzenesulfonate, p-toluenesulfonate, etc.); a salt with a basic or acidic amino acid (e.g. arginine, aspartic acid, glutamic acid, etc.); and the like, and the preferable example thereof is an acid addition salt.

In the above and subsequent descriptions of the present specification, suitable examples and illustrations of the various definitions which the present invention include within the scope thereof are explained in detail as follows.

The term "lower" is intended to mean 1 to 6 carbon atoms, preferably 1 to 4 carbon atoms, unless otherwise indicated.

Suitable "lower alkyl" may include straight or branched one, having 1 to 6 carbon atom(s), such as methyl, ethyl, propyl, isopropyl, butyl, t-butyl, pentyl, hexyl, preferably one having 1 to 4 carbon atoms, and the like, in which the most preferred one is propyl, butyl and pentyl.

Suitable "lower alkylene" is one having 1 to 6 carbon atom(s) and may include methylene, ethylene, methylmethylene, trimethylene, propylene, tetramethylene, methyltrimethylene, dimethylethylene, hexamethylene, and the like, in which the preferred one is methylene.

Suitable "esterified carboxy" may include lower alkoxycarbonyl (e.g. methoxycarbonyl, ethoxycarbonyl, etc.), and the like.

Suitable "imino-protective group" may include conventional one, and the preferable example thereof is ar(lower)alkyl such as mono-(or di- or tri-)phenyl(lower)alkyl (e.g. benzyl, benzhydryl, trityl, etc.), acyl such as N,N-di(lower)alkylsulfamoyl (e.g. N,N-dimethylsulfamoyl, etc.), lower alkanesulfonyl (e.g. mesyl, etc.), arenesulfonyl (e.g. tosyl, etc.), and the like, in which the most preferred one is trityl.

Suitable "lower alkoxy" may include straight or branched one such as methoxy, ethoxy, propoxy,

isopropoxy, butoxy, isobutoxy, tert-butoxy, pentyloxy, hexyloxy or the like, in which the preferable one is $C_1$-$C_4$ alkoxy, the more preferable one is $C_1$-$C_2$ alkoxy and the most preferable one is methoxy.

Suitable "halogen" means fluoro, chloro, bromo and iodo.

Suitable "halo(lower)alkyl" may include mono or di or trihalo(lower)alkyl such as chloroethyl, dibromoethyl, trifluoromethyl, trifluoromethylpropyl, and the like.

Suitable "acid residue" may include halogen as mentioned above, and the like.

Suitable "cyclo(lower)alkyl" is one having 3 to 6 carbon atoms and may include cyclopropyl, cyclobutyl, cyclopentyl, cyclohexyl, and the like.

Suitable "lower alkylthio" may include methylthio, ethylthio, propylthio, hexylthio, and the like.

Suitable "halo(lower)alkoxy" may include mono or di or trihalo(lower)alkoxy such as chloroethoxy, dibromoethoxy, trifluoromethoxy, trifluoroethoxy (e.g. 2,2,2-trifluoroethoxy, etc.), and the like.

Particularly, the preferred combination of $X^1$, $X^2$, $X^3$ and $X^4$ are as follows.

$X^1$ is N, and
$X^2$, $X^3$ and $X^4$ are each CH;
$X^4$ is N, and
$X^1$, $X^2$ and $X^3$ are each CH;
$X^4$ is N,
$X^1$ is $CR^4_a$ , in which $R^4_a$ is lower alkyl, and
$X^2$ and $X^3$ are each CH;
$X^4$ is N,
$X^2$ is $CR^4_b$ in which $R^4_a$ is lower alkyl or halogen, and
$X^1$ and $X^3$ are each CH;
$X^4$ is N,
$X^1$ is $CR^4_a$ in which $R^4_a$ is as defined above,
$X^2$ is $CR^4_c$ , in which $R^4_c$ is halogen, and
$X^3$ is CH;
$X^4$ is N,
$X^1$ and $X^2$ are each CH, and
$X^3$ is $CR^4_d$ , in which $R^4_d$ is halogen, lower alkoxy, halo(lower)alkoxy or hydroxy;
$X^1$ and $X^4$ are each N, and
$X^2$ and $X^3$ are each CH;
$X^2$ and $X^4$ are each N, and
$X^1$ and $X^3$ are each CH;
$X^1$ and $X^3$ are each N, and
$X^2$ and $X^4$ are each CH.

The processes for preparing the object compound (I) of the present invention are explained in detail in the following.

Process 1 :

The object compound (I) or a salt thereof can be prepared by subjecting the compound (II) to the formation reaction of a tetrazole group.

The agent to be used in the present reaction may include a conventional one which are capable of transforming a cyano group to a tetrazolyl group such as azide compound, for example, alkali metal azide (e.g., potassium azide, sodium azide etc.), tri(lower)alkyltin azide (e.g. trimethyltin azide, etc.), triaryltin azide (e.g. triphenyltin azide, etc.), or the like.

The present reaction is usually carried out in a solvent such as xylene, dioxane, chloroform, methylene chloride, 1,2-dichloroethane, tetrahydrofuran, pyridine, acetonitrile, dimethylformamide or any other solvent which does not adversely affect the reaction.

The reaction temperature is not critical and the reaction is usually carried out under warming or heating, preferably under heating.

Process 2 :

The object compound (I) or a salt thereof can be prepared by reacting the compound (III) or a salt thereof with the compound (IV) or a salt thereof.

The present reaction is usually carried out in the presence of a base such as alkyl lithium (e.g. n-butyl lithium, etc.), alkali metal hydride (e.g. sodium hydride, potassium hydride, etc.), di(lower)alkylamine (e.g. diisopropylamine, etc.), tri(lower) alkylamine (e.g. trimethylamine, triethylamine, etc.), pyridine or its derivative (e.g. picoline, lutidine, 4-dimethylaminopyridine, etc.), or the like.

The present reaction is usually carried out in a solvent such as dioxane, dimethyl sulfoxide, dimethylformamide, diethylformamide, dimethylacetamide, benzene, tetrahydrofuran, or any other solvent which does not adversely affect the reaction. In case that the base to be used is liquid, it can also be used as a solvent.

The reaction temperature is not critical and the reaction is usually carried out under cooling, at ambient temperature or under heating.

Process 3 :

The object compound (Ib) or a salt thereof can be prepared by subjecting the compound (Ia) to removal reaction of the imino-protective group.

Suitable method for this removal may include conventional one which is capable of removing an imino-protective group on a tetrazolyl group such as hydrolysis, reduction, or the like. The hydrolysis is preferably carried out in the presence of the base or an acid.

Suitable base may include, for example, an inorganic base such as alkali metal hydroxide (e.g. sodium hydroxide, potassium hydroxide, etc.), alkaline earth metal hydroxide (e.g. magnesium hydroxide, calcium hydroxide, etc.), alkali metal carbonate, (e.g. sodium carbonate, potassium carbonate, etc.), alkaline earth metal carbonate (e.g. magnesium carbonate, calcium carbonate, etc.), alkali metal bicarbonate (e.g. sodium bicarbonate, potassium bicarbonate, etc.), alkali metal acetate (e.g. sodium acetate, potassium acetate, etc.), alkaline earth metal phosphate (e.g. magnesium phosphate, calcium phosphate, etc.), alkali metal hydrogen phosphate (e.g. disodium hydrogen phosphate, dipotassium hydrogen phosphate, etc.), or the like, and an organic base such as trialkylamine (e.g. trimethylamine, triethylamine, etc.), picoline, N-methylpyrrolidine, N-methylmorpholine, 1,5-diazabicyclo[4,3,0]non-5-one, 1,4-diazabicyclo[2,2,2]octane, 1,5-diasabicyclo[5,4,0]undecene-5 or the like. The hydrolysis using a base is often carried out in water or a hydrophilic organic solvent or a mixed solvent thereof.

Suitable acid may include an organic acid (e.g. formic acid acetic acid, propionic acid, etc.) and an inorganic acid (e.g. hydrochloric acid, hydrobromic acid, sulfuric acid, etc.).

The present hydrolysis is usually carried out in an organic solvent, water or a mixed solvent thereof.

The reaction temperature is not critical, and the reaction is usually carried out at ambient temperature or under warming or heating.

Process 4 :

The object compound (I) or a salt thereof can be prepared by subjecting the compound (V) or a salt thereof to formation reaction of a imidazole group and a tetrazole group.

This reaction can be carried out in substantially the same manner as Process 1 , and therefore the reaction mode and reaction conditions (e.g. solvent, reaction temperature, etc.) of this reaction are to be referred to those as explained in Process 1 .

Process 5 :

The object compound (I) or a salt thereof can be prepared by subjecting the compound (VI) or a salt thereof to the formation reaction of a imidazole group.

This reaction can be carried out by a conventional manner which is capable of forming an imidazole group from a group of $H_2N$-CH=CH-CO- such as reflux.

The present reaction includes, within its scope, the case that the imino-protective group on $R^2$ is removed during the reaction or at the post-treating step of the present process.

The object compound (I) of the present invention can be isolated and purified in a conventional manner, for example, extraction, precipitation, fractional crystallization, recrystallisation, chromatography, and the like.

The object compound (I) thus obtained can be converted to its salt by a conventional method.

Among the starting compounds (II), (III), (IV), (V) and (VI), some of them are new and can be prepared

by the methods of Preparations mentioned below or a conventional manner.

Particularly the starting compounds (II), (IV), (V) and (VI) can be prepared by the following methods.

Method A

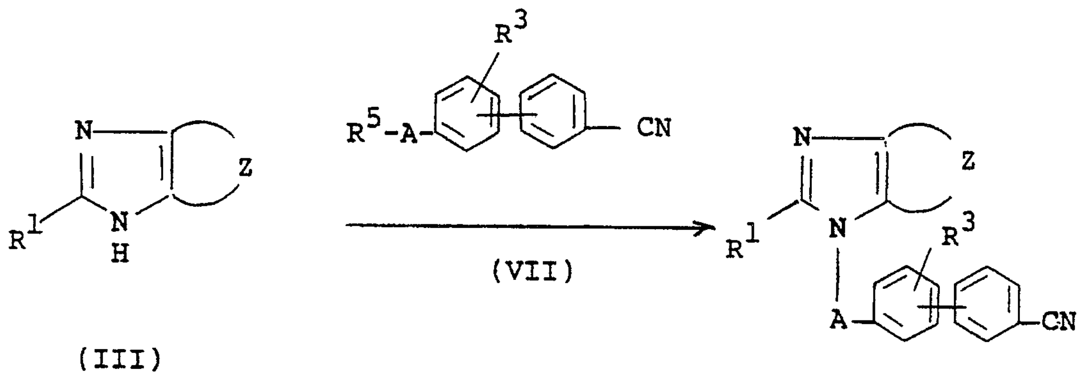


(III)
or a salt thereof

(II)

Method B

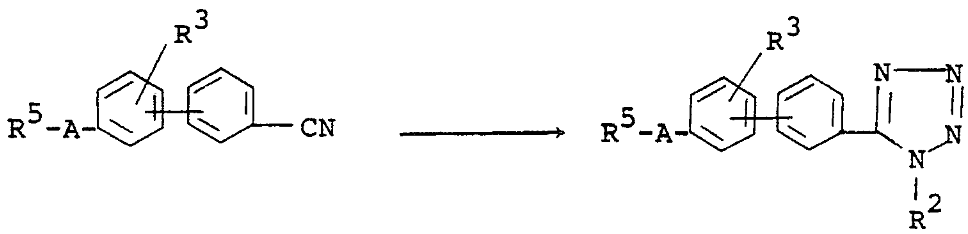

(VII)

(IV)
or a salt thereof

Method C

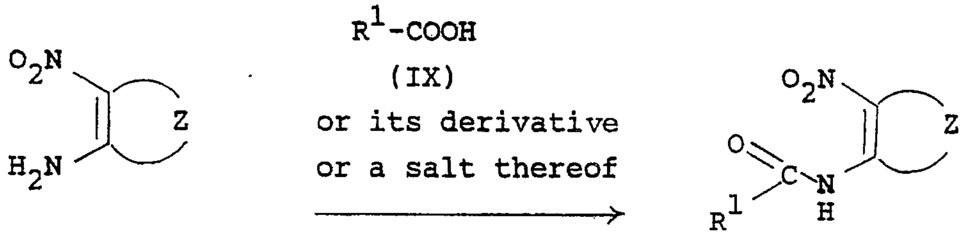


(VIII)
or a salt thereof

(X)
or a salt thereof

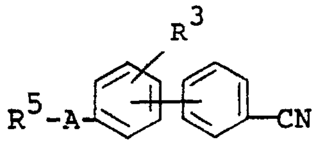

(VII)

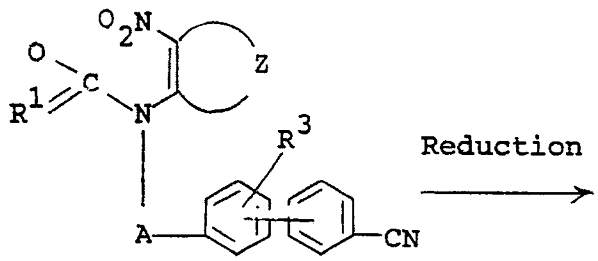


(XI)
or a salt thereof

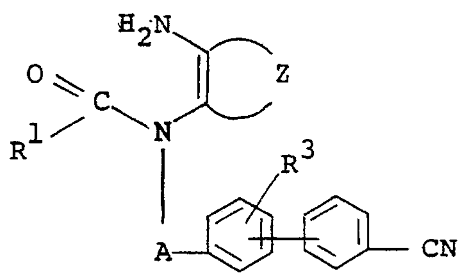

(V)
or a salt thereof

Method D

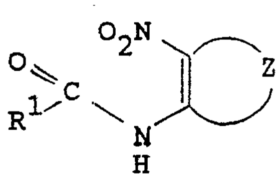

(X)
or a salt thereof

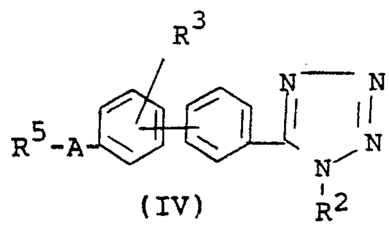

(IV)
or a salt thereof

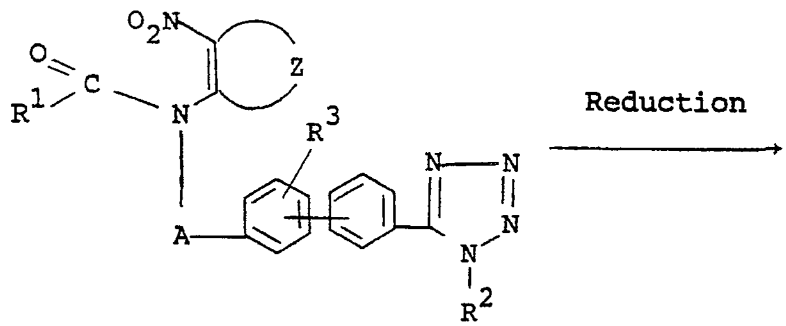


(XII)
or a salt thereof

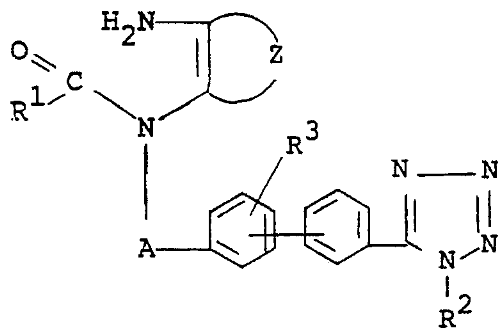

(VI)
or a salt thereof

wherein $R^1$, $R^2$, $R^3$, A and

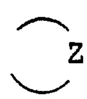

are each as defined above.

The reactions A to D can be carried out in substantially the same manner as Processes 1 to 5 or a conventional manner.

The object compounds (I) of the present invention are novel and exhibit pharmacological activities such as angiotension II antagonism, and the like, and therefore are useful as angiotension II antagonist for treating or preventing angiotension II mediated diseases, for example, hypertension (e.g. essential hypertension, renal hypertension, etc.), heart failure, and the like.

Further, it is expected that the object compounds of the present invention are useful as therapeutical and/or preventive agents for cardiopathy (e.g. angina pectoris, arrhythmia, myocardial infarction, etc.), hyperaldosteronism, cerebral vascular diseases, senile dementia, ophthalmic diseases (e.g. glaucoma, etc.), and the like; and diagnostic agents to test the renin angiotensin system.

In order to illustrate the usefulness of the object compounds (I), pharmacological activities of representative compounds of the present invention are shown below.

[1] Test compound :

① Sodium salt of 2-butyl-3-[[2′-(1H-tetrazol-5-yl)biphenyl-4-yl]methyl]-3H-imidazo[4,5-b]pyridine (hereinafter referred to as compound ① )

[2] (A) Inhibition by the antagonist of contractile response to angiotensin II in excised guinea pig ileum

Test Method :

Male guinea pigs weighing 300 g to 500 g were sacrificed by decapitation and the ileum were excised. Longitudinal strips of the ileum (length: 2 cm) were placed in a 25 ml organ bath containing Tyrode's solution of the following composition (mM): NaCl, 137; KCl, 2.7; $CaCl_2$, 1.8; $MgCl_2$, 1.1; $NaH_2PO_4$, 0.4; $NaHCO_3$, 12; Glucose, 5.6.

The bath was maintained at 37° C and bubbled with 95% $O_2$ + 5% $CO_2$. The strips was stretched with a resting force of 0.5 g, and the isometric contraction were recorded via force development transducer on an ink-writing recorder. The preparation was equilibrated in Tyrode's solution mentioned above for 30 minutes, and then exposed to atropine ($3.2 \times 10^{-7}$ g/ml). Five minutes later, the response for angiotensin II ($1 \times 10^{-8}$ g/ml) was obtained and the preparation was washed a few times. This procedure was repeated twice. After the last response for angiotensin II was obtained (control response), the preparation was washed, and the response for angiotensin II ($10^{-8}$ g/ml) was obtained in the presence of the test compound. The concentration of the test compound were $10^{-7}$, $10^{-8}$, $10^{-9}$, $10^{-10}$ M. The test compound were added 3 minutes prior to adding angiotensin II. Atropine was also added 5 minutes prior to adding angiotensin II. The inhibition of the test compound for angiotensin II contraction were expressed as a percentage change to control response, and $IC_{50}$ (M) was calculated.

| Test Result : | |
|---|---|
| Compound | $IC_{50}$ (M) |
| ① | $1.30 \times 10^{-9}$ |

(B) Effect on spontaneous hypertensive rats

Test Method :

14 to 17-week-old male spontaneous hypertensive rats with mean arterial blood pressure of about 150 mmHg, weighing 270-326 g, were used. The animals were cannulated in the left femoral artery and the mean blood pressure and heart rate were measured with a pressure-transducer. The drugs were given orally. The animals were deprived of food for about 18 hours before oral dosing. The test drugs were dissolved in water, and given in oral dose of 100 mg/kg.

Test Result :

Mean ratios of maximum decrease of blood pressure (mmHg) are shown in Table.

| Test Result : | |
|---|---|
| Compound | Effect Max (%) |
| ① | 17.6 |

For therapeutic or preventive administration, the object compound (I) of the present invention are used in the form of conventional pharmaceutical preparation which contains said compound as an active ingredient, in admixture with pharmaceutically acceptable carriers such as an organic or inorganic solid or liquid excipient which is suitable for oral, parenteral and external administration. The pharmaceutical preparation may be in solid form such as tablet, granule, powder, capsule, or liquid form such as solution, suspension, syrup, emulsion, lemonade and the like.

If needed, there may be included in the above preparations auxiliary substances, stabilizing agents, wetting agents and other commonly used additives such as lactose, citric acid, tartaric acid, stearic acid, magnesium stearate, terra alba, sucrose, corn starch, talc, gelatin, agar, pectin, peanut oil, olive oil, cacao butter, ethylene glycol, and the like.

While the dosage of the compound (I) may vary from and also depend upon the age, conditions of the patient, a kind of diseases or conditions, a kind of the compound (I) to be applied, etc. In general amounts between 0.01 mg and about 500 mg or even more per day may be administered to a patient. An average single dose of about 0.05 mg, 0.1 mg, 0.25 mg, 0.5 mg, 1 mg, 20 mg, 50 mg, 100 mg of the object compound (I) of the present invention may be used in treating diseases.

The following Preparations and Examples are given for the purpose of illustrating the present invention.

## Preparation 1

To a solution of 2-butylimidazo[4,5-b]pyridine (650 mg) in dimethyl sulfoxide (15 ml) was added sodium hydride (148 mg, 60% in oil). After being stirred at room temperature for 30 minutes, 4′-bromomethyl-2-cyanobiphenyl (1.00 g) was added to the solution and the mixture was stirred at room temperature overnight. The reaction mixture was diluted with ethyl acetate and washed successively with water and brine and dried over magnesium sulfate. The solvent was evaporated in vacuo and the residue was chromatographed on a silica gel column with a mixture of chloroform and methanol (50:1) as an eluent to give 2-butyl-3-[(2′-cyanobiphenyl-4-yl)methyl]-3H-imidazo[4,5-b]pyridine (736 mg) as desired compound.

mp : 94-95°C

NMR ($CDCl_3$, δ) : 0.93 (3H, t, J=7.5Hz), 1.43 (2H, m), 1.85 (2H, m), 2.88 (2H, t, J=7.5Hz), 5.57 (2H, s), 7.20-7.30 (3H, m), 7.40-7.55 (4H, m), 7.64 (1H, td, J=8, 1Hz), 7.77 (1H, d, J=7.5Hz), 8.06 (1H, dd, J=7.5, 1Hz), 8.37 (1H, dd, J=7.5, 1Hz)

## Preparation 2

To a solution of 8-butylpurine (654 mg) in dimethyl sulfoxide (15 ml) was added sodium hydride (148 mg, 60% in oil). After being stirred at room temperature for 30 minutes, 4′-bromomethyl-2-cyanobiphenyl (1.00 g) was added to the solution and the solution was stirred at room temperature overnight. The reaction mixture was diluted with ethyl acetate and washed successively with water and brine and dried over magnesium sulfate. The solvent was evaporated in vacuo to give red-brownish residue, which was chromatographed on a silica gel column with a mixture of chloroform and methanol (50:1) as an eluent to give 8-butyl-9-[(2′-cyanobiphenyl-4-yl)methyl]purine (734 mg) as desired compound.

mp : 134-135°C

NMR ($CDCl_3$, δ) : 0.94 (3H, t, J=7.5Hz), 1.45 (2H, m), 1.86 (2H, m), 2.89 (2H, t, J=7.5Hz), 5.53 (2H, s), 7.28 (2H, d, J=9Hz), 7.40-7.80 (6H, m), 8.98 (1H, s), 9.09 (1H, s)

## Preparation 3

A mixture of 2,3-naphthalenediamine (4 g) and valeric acid (9.3 ml) in 4N-hydrochloric acid (30 ml) was refluxed for 63 hours, and concentrated in vacuo. The residue was dissolved in water and the aqueous layer was adjusted to pH 7 with aqueous 1N-sodium hydroxide solution at 0°C. The separated oil was extracted with ethyl acetate. The organic layer was washed with aqueous 1N-sodium hydroxide solution, water and brine in turn and dried over magnesium sulfate. The solvent was distilled by evaporation and the residue was solidified from a mixture of ethanol and water to give 2-butyl-1H-naphth[2,3-d]imidazole (4.3 g).

mp : 180-184.5°C

NMR ($CDCl_3$, δ) : 0.91 (3H, t, J=7.5Hz), 1.33-1.57 (2H, m), 1.82-2.02 (2H, m), 3.02 (2H, t, J=7.5Hz), 6.23-6.78 (1H, br s), 7.40 (2H, dd, J=6.0Hz and 4.0Hz), 7.91 (2H, dd, J=6.0Hz and 4.0Hz), 8.01 (2H, s)

Preparation 4

The following compound was obtained according to a similar manner to that of Preparation 3.

2-Butyl-1H-naphth[1,2-d]imidazole

NMR ($CDCl_3$, $\delta$) : 0.88 (3H, t, J=7.5Hz), 1.29-1.49 (2H, m), 1.76-1.93 (2H, m), 3.00 (2H, t, J=7.5Hz), 4.67-5.29 (1H, br s), 7.39-7.58 (2H, m), 7.66 (2H, s), 7.90-7.98 (1H, m), 8.34-8.42 (1H, m)

Preparation 5

The following desired compounds were obtained according to a similar manner to that of Preparation 1 or 2.

(1) 3-[2'-Cyanobiphenyl-4-yl)methyl]-2-pentyl-3H-imidazo[4,5-b)pyridine

NMR ($CDCl_3$, $\delta$) : 0.86 (3H, t, J=7.5Hz), 1.25-1.5 (4H, m), 1.80-2.00 (2H, m), 2.85 (2H, t, J=7.5Hz), 5.56 (2H, s), 7.2-7.3 (3H, m), 7.40-7.55 (4H, m), 7.63 (1H, m), 7.77 (1H, dd, J=8.5Hz and 1Hz), 8.04 (1H, dd, J=8.5Hz and 1Hz), 8.38 (1H, dd, J=5Hz and 1Hz)

(2) 3-[(2'-Cyanobiphenyl-4-yl)methyl]-2-propyl-3H-imidazo[4,5-b]pyridine

NMR ($CDCl_3$, $\delta$) : 1.01 (3H, t, J=7.5Hz), 1.90 (2H, m), 2.83 (2H, t, J=7.5Hz), 5.59 (2H, s), 7.15-7.25 (3H, m), 7.40-7.55 (4H, m), 7.62 (1H, m), 7.76 (1H, d, J=8Hz), 8.03 (1H, dd, J=8Hz and 1Hz), 8.47 (1H, dd, J=5Hz and 1Hz)

(3) 2-Butyl-1-[(2'-cyanobiphenyl-4-yl)methyl]-1H-naphth[1,2-d]imidazole

NMR ($CDCl_3$, $\delta$) : 0.93 (3H, t, J=7.5Hz), 1.36-1.58 (2H, m), 1.74-1.93 (2H, m), 2.99 (2H, t, J=8.0Hz), 5.52 (2H, s), 7.13 (2H, d, J=9.0Hz), 7.31-7.82 (10H, m), 7.91 (1H, d, J=8.5Hz), 8.69 (1H, d, J=8.5Hz)

(4) 2-Butyl-1-[(2'-cyanobiphenyl-4-yl)methyl]-1H-naphth[2,3-d]imidazole

NMR ($CDCl_3$, $\delta$) : 0.97 (3H, t, J=7.0Hz), 1.38-1.60 (2H, m), 1.81-2.01 (2H, m), 2.93 (2H, t, J=8.0Hz), 5.50 (2H, s), 7.19 (2H, d, J=9Hz), 7.34-8.08 (11H, m), 8.26 (1H, s)

Preparation 6

A solution of 1.5M butyllithium in hexane (25 ml) in tetrahydrofuran (25 ml) was cooled at -78°C by using dry ice-acetone bath under nitrogen atmosphere and a solution of 4-bromotoluene (6.40 g) in tetrahydrofuran (25 ml) was added dropwise to the solution. After being stirred at -78°C for 1 hour, a solution of freshly fused zinc chloride (5.15 g) in tetrahydrofuran (25 ml) was added in a slow stream via cannula and the resulting mixture was allowed to warm to room temperature. The mixture was stirred at room temperature for 1 hour and the resulting solution was added to a solution of 2-bromobenzonitrile (4.55 g) and tetrakis(triphenylphosphine)palladium(0) (400 mg) in tetrahydrofuran (25 ml) at room temperature. The solution was stirred at room temperature overnight. The solution was diluted with ethyl acetate (300 ml) and washed successively with 1N-hydrochloric acid, water, and brine. The organic solution was dried over magnesium sulfate and the solvent was evaporated in vacuo. The residue was purified by silica gel column chromatography with a mixture of ethyl acetate and n-hexane (1:20) as eluent to give 2-cyano-4'-methylbiphenyl (4.43 g) as white powders.

mp : 44-47°C

NMR ($CDCl_3$, $\delta$) : 2.42 (3H, s), 7.25-7.35 (2H, m), 7.35-7.5 (4H, m), 7.62 (1H, dt, J=1, 7.5Hz), 7.85 (1H, dd, J=1, 7.5Hz)

Preparation 7

A mixture of 2-amino-3-nitropyridine (25 g) and N,N-dimethylaniline (46 ml) was heated at 110°C under nitrogen atmosphere. To the solution was added valeryl chloride (22.4 ml) and the mixture was stirred at 110°C for 2 hours. After being cooled to room temperature, ethyl acetate (400 ml) and water (400 ml) were added to the reaction mixture. The organic layer was separated and washed successively with water (400 ml) and brine (400 ml). The solution was dried over magnesium sulfate and the solvent was evaporated in vacuo. The residue was purified with silica gel column chromatography ($CHCl_3$-MeOH, 30:1) to give crude solid (35.5 g). The solid was recrystallized from isopropyl ether to give 3-nitro-2-valerylaminopyridine (31.8 g).

mp : 114-115°C

NMR ($CDCl_3$, δ) : 0.98 (3H, t, J=7.5Hz), 1.32-1.55 (2H, m), 1.67-1.85 (2H, m), 2.66 (2H, t, J=7.5Hz), 7.22 (1H, dd, J=9, 4.5Hz), 8.49 (1H, dd, J=9, 1Hz), 8.69 (1H, dd, J=4.5, 1Hz), 9.87 (1H, br s)

Preparation 8

To a suspension of sodium hydride (1.7 g, 60% oil dispersion) in dimethyl sulfoxide (100 ml) was added a solution of 3-nitro-2-valerylaminopyridine (9.4 g) in dimethyl sulfoxide (100 ml) at room temperature under nitrogen atmosphere. After being stirred at room temperature for 1 hour, 4′-bromomethyl-2-cyanobiphenyl (11.4 g) in dimethyl sulfoxide (100 ml) was added dropwise to the solution and the mixture was stirred at room temperature for 1.5 hours. The reaction mixture was poured into a mixture of ethyl acetate (500 ml) and ice water (300 ml) and extracted with ethyl acetate. The organic layer was washed successively with water (300 ml x 2) and brine (300 ml) and dried over magnesium sulfate. The solvent was evaporated in vacuo and the residue was purified with silica gel column chromatography (ethylacetate-n-hexane, 1:2) to give 2-[N-[(2′-cyanobiphenyl-4-yl)methyl]-N-valerylamino]-3-nitropyridine (15.2 g) as an oil.
NMR ($CDCl_3$, δ) : 0.85 (3H, t, J=7.5Hz), 1.19-1.42 (2H, m), 1.56-1.79 (2H, m), 2.49 (2H, br), 5.41 (2H, br), 7.18-7.71 (8H, m), 8.28 (1H, d, J=7.5Hz), 8.52-8.87 (2H, m)

Preparation 9

A solution of 2-[N-[(2′-cyanobiphenyl-4-yl)methyl]-N-valerylamino]-3-nitropyridine (15.1 g) and iron powder (20 g) in a mixture of acetic acid (25 ml) and ethanol (200 ml) was stirred at 80 °C for 1 hour under nitrogen atmosphere. After being cooled to room temperature, the reaction mixture was filtered through Celite and the filtrate was evaporated in vacuo. Ethyl acetate (700 ml) and saturated aqueous sodium hydrogencarbonate (600 ml) were added to the residue and the resulting suspension was filtered through Celite. The organic layer of the filtrate was separated and washed brine (300 ml), dried over magnesium sulfate. The solvent was evaporated in vacuo and the residue was purified with silica gel column chromatography (ethyl acetate-n-hexane, 1:2) to give 2-butyl-3-[(2′-cyanobiphenyl-4-yl)methyl]-3H-imidazo-[4,5-b]pyridine (11.4 g).

Preparation 10

A solution of 3-amino-2-[(2′-cyanobiphenyl-4-yl)methylamino]pyridine (400 mg) and potassium xanthogenate (427 mg) in a mixture of ethanol (10 ml) and water (3 ml) was refluxed for 4 hours and cooled to room temperature. The reaction mixture was diluted with chloroform (40 ml) and the solution was washed successively with water and brine, dried over magnesium sulfate. The solvent was evaporated in vacuo and the residue was solidified with ether to give 3-[(2′-cyanobiphenyl-4-yl)methyl]-2-mercapto-3H-imidazo[4,5-b]pyridine (318 mg).
mp : 246-247 °C
NMR (DMSO-$d_6$, δ) : 3.32 (1H, s), 5.57 (2H, s), 7.25 (1H, dd, J=5, 9Hz), 7.50-7.70 (7H, m), 7.77 (1H, dt, J=1, 9Hz), 7.94 (1H, d, J=9Hz), 8.22 (1H, dd, J=1, 5Hz)

Preparation 11

To a solution of 3-[(2′-cyanobiphenyl-4-yl)methyl]-2-mercapto-3H-imidazo[4,5-b]pyridine (300 mg) in dimethyl sulfoxide (5 ml) was added sodium hydride (35 mg, 60% oil dispersion) and the mixture was stirred at room temperature for 25 minutes. 1-Iodopropane (150 mg) was added to the solution and the mixture was stirred at room temperature for 3 hours. The reaction mixture was diluted with ethyl acetate and washed successively with water and brine. The organic solution was dried over magnesium sulfate and the solvent was evaporated in vacuo. The residue was purified with silica gel column chromatography to give 3-[(2′-cyanobiphenyl-4-yl)methyl]-2-propylthio-3H-imidazo[4,5-b]pyridine (391 mg) as an oil.
NMR ($CDCl_3$, δ) : 1.08 (3H, t, J=7.5Hz), 1.81 (2H, m), 3.40 (2H, t, J=7.5Hz), 5.49 (2H, s), 7.20 (1H, dd, J=5, 9Hz), 7.40-7.55 (6H, m), 7.63 (1H, dt, J=1, 9Hz), 7.75 (1H, dd, J=1,9Hz), 7.94 (1H, dd, J=1, 9Hz), 8.30 (1H, dd, J=1, 5Hz)

Preparation 12

The following desired compounds were obtained according to a similar manner to that of Preparation 1 or 2.

(1) 2-Butyl-1-[(2′-cyanobiphenyl-4-yl)methyl]-1H-imidazo[4,5-b]pyrazine

NMR ($CDCl_3$, δ) : 0.92 (3H, t, J=7.5Hz), 1.45 (2H, m), 1.88 (2H, m), 2.91 (2H, t, J=7.5Hz), 5.56 (2H, s), 7.28 (2H, d, J=8Hz), 7.40-7.60 (4H, m), 7.62 (1H, dt, J=1, 8Hz), 7.77 (1H, dd, J=1, 8Hz), 8.30 (1H, d, J=2.5Hz), 8.50 (1H, d, J=2.5Hz)

(2) 2-Butyl-3-[1-(2′-cyanobiphenyl-4-yl)ethyl]-3H-imidazo[4,5-b]pyridine

NMR ($CDCl_3$, δ) : 0.89 (3H, t, J=7.5Hz), 1.38 (2H, m), 1.79 (2H, m), 2.15 (3H, d, J=7Hz), 2.79 (2H, m), 6.18 (1H, q, J=7Hz), 7.22 (1H, dd, J=9, 5Hz), 7.40-7.55 (6H, m), 7.64 (1H, dt, J=1, 8Hz), 7.78 (1H, dd, J=1, 8Hz), 8.03 (1H, dd, J=1, 8Hz), 8.35 (1H, dd, J=1,5Hz)

(3) 3-[(2′-Cyanobiphenyl-4-yl)methyl]-2-[2-(trifluoromethyl)propyl]-3H-imidazo[4,5-b]pyridine

NMR ($CDCl_3$, δ) : 1.19 (3H, d, J=7Hz), 2.86 (1H, dd, J=15, 9.5Hz), 3.10 (1H, m), 3.23 (1H, dd, J=15, 4Hz), 5.51 (1H, d, J=15Hz), 5.68 (1H, d, J=15Hz), 7.25-7.35 (3H, m), 7.40-7.55 (4H, m), 7.63 (1H, dt, J=1, 8Hz), 7.75 (1H, dd, J=1, 8Hz), 8.07 (1H, dd, J=8, 1Hz), 8.43 (1H, dd, J=5, 1Hz)

Preparation 13

The following desired compounds were obtained according to a similar manner to that of Preparation 3.

(1) 2-Butylimidazo[4,5-b]pyrazine

mp : 219-223 °C

NMR (DMSO-$d_6$, δ) : 0.98 (3H, t, J=7.5Hz), 1.45 (2H, m), 1.88 (2H, m), 2.97 (2H, t, J=7.5Hz), 8.32 (2H, s)

(2) 2-[2-(Trifluoromethyl)propyl]-3H-imidazo[4,5-b]pyridine

mp : 154-155 °C

NMR ($CDCl_3$, δ) : 1.26 (3H, d, J=6Hz), 3.04 (2H, m), 3.48 (1H, m), 7.32 (1H, dd, J=8, 5Hz), 8.11 (1H, dd, J=8, 1Hz), 8.36 (1H, dd, J=5, 1Hz)

Preparation 14

The following compound was obtained according to a similar manner to that of Preparation 6.

2-Cyano-4′-ethylbiphenyl

NMR ($CDCl_3$, δ) : 1.29 (3H, t, J=7.5Hz), 2.72 (2H, q, J=7.5Hz), 7.32 (2H, d, J=8Hz), 7.41 (1H, dd, J=1, 8Hz), 7.45-7.55 (3H, m), 7.63 (1H, dt, J=1, 8Hz), 7.78 (1H, dd, J=1, 8Hz)

Preparation 15

The following compounds were obtained according to a similar manner to that of Preparation 7.

(1) 4-Methyl-3-nitro-2-valerylaminopyridine

mp : 114-116 °C

NMR ($CDCl_3$, δ) : 0.82 (3H, t, J=7.5Hz), 1.37 (2H, m), 1.70 (2H, m), 2.45 (2H, t, J=7.5Hz), 2.50 (1H, s), 7.10 (1H, d, J=5Hz), 8.35 (1H, d, J=4Hz), 8.53 (1H, br)

(2) 6-Chloro-3-nitro-2-valerylaminopyridine

mp : 101-102 °C

NMR ($CDCl_3$, δ) : 0.96 (3H, t, J=7.5Hz), 1.47 (2H, m), 1.12 (2H, m), 2.72 (2H, t, J=7.5Hz), 7.18 (1H, d, J=9Hz), 8.43 (1H, d, J=9Hz)

(3) 6-Methoxy-3-Nitro-2-valerylaminopyridine

mp : 62-64 °C

NMR ($CDCl_3$, δ) : 0.97 (3H, t, J=7.5Hz), 1.43 (2H, m), 1.76 (2H, m), 2.79 (2H, t, J=7.5Hz), 4.06 (3H, s), 6.51 (1H, d, J=9Hz), 8.42 (1H, d, J=9Hz)

(4) 3-Nitro-6-(2,2,2-trifluoroethoxy)-2-valerylaminopyridine

mp : 61-62 °C

NMR ($CDCl_3$, δ) : 0.98 (3H, t, J=7.5Hz), 1.44 (2H, m), 1.78 (2H, m), 2.62 (2H, t, J=7.5Hz), 4.91 (2H, q, J=8.5Hz), 6.66 (1H, d, J=9Hz), 8.52 (1H, d, J=9Hz)

(5) 6-Allyloxy-3-nitro-2-valerylaminopyridine
NMR ($CDCl_3$, δ) : 0.96 (3H, t, J=7.5Hz), 1.42 (2H, m), 1.76 (2H, m), 2.62 (2H, t, J=7.5Hz), 4.96 (2H, dt, J=5.5, 1Hz), 5.32 (1H, dd, J=10, 1Hz), 5.43 (1H, dd, J=16, 1Hz), 6.08 (1H, m), 6.52 (1H, d, J=9Hz), 8.44 (1H, d, J=9Hz)
(6) 2-Cyclopropylcarbonylamino-3-nitropyridine
mp : 152-163 °C
NMR ($CDCl_3$, δ) : 0.92-1.05 (2H, m), 1.16-1.28 (2H, m), 1.89-2.06 (1H, m), 7.22 (1H, dd, J=9.0, 5.0Hz), 8.48 (1H, dd, J=9.0, 1.0Hz), 8.70 (1H, dd, J=5.0, 1.0Hz)
(7) 2-Cyclohexylcarbonylamino-3-nitropyridine
mp : 169-174 °C
NMR ($CDCl_3$, δ) : 1.21-2.13 (10H, m), 2.41-2.59 (1H, m), 7.23 (1H, dd, J=8.5, 4.5Hz), 8.49 (1H, dd, J=8.5, 1.0Hz), 8.73 (1H, dd, J=4.5, 1.0Hz), 9.85 (1H, br s)

## Preparation 16

The following compounds were obtained according to a similar manner to that of Preparation 8.
(1) 2-[N-[(2′-Cyanobiphenyl-4-yl)methyl]-N-valerylamino]-4-methyl-3-nitropyridine
NMR ($CDCl_3$, δ) : 0.86 (3H, t, J=7.5Hz), 1.29 (2H, m), 1.64 (2H, m), 2.13 (2H, br), 2.43 (3H, s), 5.22 (2H, br s), 7.40-7.60 (7H, m), 7.62 (1H, dt, J=1, 8Hz), 7.78 (1H, dt, J=1, 8Hz), 8.50 (1H, d, J=5Hz)
(2) 6-Chloro-2-(N-[(2′-cyanobiphenyl-4-yl)methyl]-N-valerylamino]-3-nitropyridine
NMR ($CDCl_3$, δ) : 0.86 (3H, t, J=7.5Hz), 1.31 (2H, m), 1.63 (2H, m), 2.47 (2H, br), 5.35 (2H, br s), 7.32 (1H, d, J=9Hz), 7.40-7.70 (7H, m), 7.78 (1H, d, J=8Hz), 8.22 (1H, d, J=9Hz)
(3) 2-[N-[(2′-Cyanobiphenyl-4-yl)methyl]-N-valerylamino]-6-methoxy-3-nitropyridine
NMR ($CDCl_3$, δ) : 0.84 (3H, t, J=7.5Hz), 1.30 (2H, m), 1.68 (2H, m), 2.32 (2H, br), 3.82 (3H, s), 5.30 (2H, br s), 6.72 (1H, d, J=9Hz), 7.40-7.55 (6H, m), 7.63 (1H, dt, J=1, 8Hz), 7.77 (1H, dt, J=1, 8Hz), 8.28 (1H, d, J=9Hz)
(4) 6-Chloro-3-nitro-2-[N-[[2′-(1-trityl-1H-tetrazol-5-yl)biphenyl-4-yl]methyl]-N-valerylamino]pyridine
NMR ($CDCl_3$, δ) : 0.81 (3H, t, J=7.5Hz), 1.27 (2H, m), 1.58 (2H, m), 2.35 (2H, m), 5.15 (2H, br s), 6.85-6.95 (6H, m), 7.10-7.50 (17H, m), 7.90 (1H, m), 8.11 (1H, d, J=9Hz)
(5) 2-[N-[(2′-Cyanobiphenyl-4-yl)methyl]-N-valerylamino]-3-nitro-6-(2,2,2-trifluoroethoxy)pyridine
NMR ($CDCl_3$, δ) : 0.85 (3H, t, J=7.5Hz), 1.29 (2H, m), 1.67 (2H, m), 2.41 (2H, m), 4.56 (2H, m), 5.28 (2H, br s), 6.87 (1H, d, J=9Hz), 7.40-7.60 (6H, m), 7.67 (1H, dt, J=8, 1Hz), 7.78 (1H, dd, J=8, 1Hz), 8.35 (1H, d, J=9Hz)
(6) 6-Allyloxy-2-[N-[(2′-cyanobiphenyl-4-yl)methyl]-N-valerylamino]-3-nitropyridine
NMR ($CDCl_3$, δ) : 0.87 (3H, t, J=7.5Hz), 1.28 (2H, m), 1.71 (2H, m), 2.41 (2H, m), 4.71 (2H, d, J=5.5Hz), 5.20-5.35 (4H, m), 5.94 (1H, m), 6.76 (1H, d, J=9Hz), 7.40-7.80 (8H, m), 8.28 (1H, d, J=9Hz)
(7) 2-[N-[(2′-Cyanobiphenyl-4-yl)methyl]-N-cyclopropylcarbonylamino]-3-nitropyridine
NMR ($CDCl_3$, δ) : 0.78 (2H, br s), 1.11 (2H, br s), 1.83 (1H, br s), 5.62 (2H, br s), 7.22-7.83 (9H, m), 8.18-8.40 (1H, m), 8.57-8.83 (1H, m)
(8) 2-[N-[(2′-Cyanobiphenyl-4-yl)methyl]-N-cyclohexylcarbonylamino]-3-nitropyridine
NMR ($CDCl_3$, δ) : 0.82-1.95 (10H, m), 2.50-2.74 (1H, m), 4.99 (0.6H, br s), 5.43 (1.4H, br s), 7.21-7.84 (9H, m), 8.25 (1H, d, J=7.5Hz), 8.53-8.83 (1H, m)
(9) 5-Bromo-2-[N-[(2′-cyanobiphenyl-4-yl)methyl]-N-valerylamino]-3-nitropyridine
NMR ($CDCl_3$, δ) : 0.86 (3H, t, J=7.5Hz), 1.20-1.39 (2H, m), 1.54-1.75 (2H, m), 2.49 (2H, br s), 5.30 (0.8H, s), 5.38 (1.2H, br s), 7.39-7.60 (6H, m), 7.66 (1H, dt, J=1.0, 7.5Hz), 7.78 (1H, dd, J=7.5, 1.0Hz), 8.39 (1H, d, J=1.0Hz), 8.58-8.74 (1H, m)
(10) 2-(N-[(2′-Cyanobiphenyl-4-yl)methyl]-N-valerylamino]-5-methyl-3-nitropyridine
NMR ($CDCl_3$, δ) : 0.78-0.93 (3H, m), 1.15-1.42 (2H, m), 1.55-1.78 (2H, m), 2.02-2.19 (1H, m), 2.39-2.56 (4H, m), 4.98 (0.8H, br s), 5.36 (1.2H, br s), 7.25-7.82 (8H, m), 8.06 (1H, s), 8.39-8.62 (1H, m)
(11) 5-Bromo-2-[N-[(2′-cyanobiphenyl-4-yl)methyl]-N-valerylamino]-4-methyl-3-nitropyridine
NMR ($CDCl_3$, δ) : 0.85 (3H, t, J=7.5Hz), 1.18-1.39 (2H, m), 1.52-1.71 (2H, m), 1.99-2.22 (2H, m), 2.43 (3H, s), 4.61-5.29 (2H, m), 7.29-7.55 (6H, m), 7.62 (1H, dt, J=1.0, 7.5Hz), 7.74 (1H, dd, J=7.5, 1.0Hz), 8.68 (1H, s)

## Preparation 17

The following compounds were obtained according to a similar manner to that of Preparation 9.

(1) 2-Butyl-3-[(2′-cyanobiphenyl-4-yl)methyl]-7-methyl-3H-imidazo[4,5-6]pyridine

NMR ($CDCl_3$, δ) : 0.90 (3H, t, J=7.5Hz), 1.42 (2H, m), 1.73 (2H, m), 2.69 (3H, s), 2.88 (2H, t, J=7.5Hz), 5.56 (2H, s), 7.06 (1H, dd, J=1, 5Hz), 7.23 (2H, d, J=8Hz), 7.40-7.55 (4H, m), 7.63 (1H, dt, J=1, 8Hz), 7.75 (1H, dd, J=1, 8Hz), 8.23 (1H, d, J=5Hz)

(2) 2-Butyl-5-chloro-3-[(2′-cyanobiphenyl-4-yl)methyl]-3H-imidazo[4,5-b]pyridine

mp : 109-110 °C

NMR ($CDCl_3$, δ) : 0.90 (3H, t, J=7.5Hz), 1.41 (2H, m), 1.81 (2H, m), 2.82 (2H, t, J=7.5Hz), 5.53 (2H, s), 7.26 (1H, d, J=9Hz), 7.28 (1H, d, J=9Hz), 7.40-7.60 (5H, m), 7.64 (1H, dt, J=1, 8Hz), 7.77 (1H, dd, J=1, 8Hz), 7.97 (1H, d, J=9Hz)

(3) 2-Butyl-3-[(2-cyanobiphenyl-4-yl)methyl]-5-methoxy-3H-imidazo]-4,5-b]pyridine

mp : 89-91 °C

NMR ($CDCl_3$, δ) : 0.91 (3H, t, J=7.5Hz), 1.41 (2H, m), 1.79 (2H, m), 2.81 (2H, t, J=7.5Hz), 3.98 (3H, s), 5.46 (2H, s), 6.68 (1H, d, J=9Hz), 7.32 (2H, d, J=8Hz), 7.40-7.55 (4H, m), 7.64 (1H, dt, J=1, 8Hz), 7.77 (1H, dd, J=1, 8Hz), 7.91 (1H, d, J=9Hz)

(4) 2-Butyl-5-(2,2,2-trifluoroethoxy)-3-[(2′-cyanobipheny-4-yl)methyl]-3H-imidazo[4,5-b]pyridine

NMR ($CDCl_3$, δ) : 0.91 (3H, t, J=7.5Hz), 1.40 (2H, m), 1.79 (2H, m), 2.79 (2H, t, J=7.5Hz), 4.79 (2H, q, J=9Hz), 5.44 (2H, s), 6.80 (1H, d, J=9Hz), 7.27 (2H, d, J=5Hz), 7.40-7.55 (4H, m), 7.65 (1H, dt, J=8, 1Hz), 7.78 (1H, dd, J=8, 1Hz), 7.98 (1H, d, J=9Hz)

(5) 5-Allyloxy-2-butyl-3-[(2′-cyanobiphenyl-4-yl)methyl]-3H-imidazo[4,5-b]pyridine

NMR ($CDCl_3$, δ) : 0.90 (3H, t, J=7.5Hz), 1.42 (2H, m), 1.82 (2H, m), 2.82 (1H, t, J=7.5Hz), 4.89 (2H, dt, J=5.5, 1Hz), 5.26 (1H, m), 5.40 (1H, m), 5.44 (2H, s), 6.60 (1H, m), 6.72 (1H, d, J=9Hz), 7.32 (2H, d, J=8Hz), 7.40-7.55 (4H, m), 7.64 (1H, dt, J=1, 8Hz), 7.78 (1H, dd, J=8, 1Hz), 7.93 (1H, d, J=9Hz)

(6) 3-[(2′-Cyanobiphenyl-4-yl)methyl]-2-cyclopropyl-3H-imidazo[4,5-b]pyridine

NMR ($CDCl_3$, δ) : 1.05-1.17 (2H, m), 1.21-1.33 (2H, m), 1.91-2.08 (1H, m), 5.69 (2H, s), 7.21 (1H, dd, J=9.0, 5.0Hz), 7.35 (2H, d, J=9.0Hz), 7.38-7.57 (4H, m), 7.63 (1H, dt, J=1.0, 7.5Hz), 7.76 (1H, dd, J=7.5, 1.0Hz), 7.95 (1H, dd, J=9.0, 1.0Hz), 8.32 (1H, dd, J=5.0, 1.0Hz)

(7) 3-[(2′-Cyanobiphenyl-4-yl)methyl]-2-cyclohexyl-3H-imidazo[4,5-b]pyridine

NMR ($CDCl_3$, δ) : 1.19-1.97 (10H, m), 2.75-2.94 (1H, m), 5.59 (2H, s), 7.23 (1H, dd, J=8.0, 5.0Hz), 7.23-7.32 (2H, m), 7.38-7.57 (4H, m), 7.62 (1H, dt, J=1.0, 7.5Hz), 7.75 (1H, dd, J=7.5, 1.0Hz), 8.05 (1H, dd, J=8.0, 1.0Hz), 8.37 (1H, dd, J=5.0, 1.0Hz)

(8) 6-Bromo-2-butyl-3-[(2′-cyanobiphenyl-4-yl)methyl]-3H-imidazo[4,5-b]pyridine

NMR ($CDCl_3$, δ) : 0.92 (3H, t, J=7.5Hz), 1.32-1.52 (2H, m), 1.73-1.91 (2H, m), 2.87 (2H, t, J=7.5Hz), 5.53 (2H, s), 7.26 (2H, d, J=9.0Hz), 740-7.58 (4H, m), 7.64 (1H, dt, J=1.0, 8.5Hz), 7.76 (1H, dd, J=8.5, 1.0Hz), 8.16 (1H, d, J=1.0Hz), 8.41 (1H, d, J=1.0Hz)

(9) 2-Butyl-3-[(2′-cyanobiphenyl-4-yl)methyl]-6-methyl-3H-imidazo[4,5-b]pyridine

NMR ($CDCl_3$, δ) : 0.91 (3H, t, J=7.5Hz), 1.31-1.52 (2H, m), 1.72-1.90 (2H, m), 2.48 (3H, s), 2.85 (2H, t, J=7.5Hz), 5.54 (2H, s), 7.25 (2H, d, J=9.0Hz), 7.51 (2H, d, J=9.0Hz), 7.49-7.55 (2H, m), 7.62 (1H, dt, J=1.0, 7.5Hz), 7.76 (1H, dd, J=7.5, 1.0Hz), 7.84 (1H, d, J=1.0Hz), 8.20 (1H, d, J=1.0Hz)

(10) 6-Bromo-2-butyl-3-[(2′-cyanobiphenyl-4-yl)methyl]-7-methyl-3H-imidazo[4,5-b]pyridine

NMR ($CDCl_3$, δ) : 0.91 (3H, t, J=7.5Hz), 1.31-1.52 (2H, m), 1.67-1.84 (2H, m), 2.74 (3H, s), 2.87 (2H, t, J=7.5Hz), 5.52 (2H, s), 7.24 (2H, d, J=9.0Hz), 7.50 (2H, d, J=9.0Hz), 7.39-7.55 (2H, m), 7.63 (1H, dt, J=1.0, 7.5Hz), 7.76 (1H, dd, J=7.5, 1.0Hz), 8.40 (1H, s)

## Preparation 18

The following compound was obtained according to a similar manner to that of Preparation 11.

3-[(2′-Cyanobiphenyl-4-yl)methyl]-2-ethylthio-3H-imidazo[4,5-b]pyridine

NMR ($CDCl_3$, δ) : 1.43 (3H, t, J=7.5Hz), 3.43 (3H, q, J=7.5Hz), 5.49 (2H, s), 7.22 (1H, dd, J=5, 9Hz), 7.40-7.55 (6H, m), 7.63 (1H, dt, J=1, 8Hz), 7.75 (1H, dd, J=1, 8Hz), 7.98 (1H, dd, J=1, 8Hz), 8.32 (1H, dd, J=1, 5Hz)

## Preparation 19

A mixture of 2-amino-6-chloro-3-nitropyridine (1.73 g), allyl alcohol (1.16 g), and potassium tert-butoxide (1.36 g) in dioxane (50 ml) was stirred at ambient temperature for 4 hours and then at 50 °C for 4 hours.

The mixture was diluted with ethyl acetate and washed with water. The organic layer was dried and concentrated in vacuo. The residue was chromatographed on silica gel (elution by chloroform) to give 6-allyloxy-2-amino-3-nitropyridine (1.60 g) as crystals.

mp : 135-138 °C

NMR ($CD_3OD$, δ) : 4.83 (2H, dt, J=5.5, 1Hz), 5.81 (1H, m), 5.90 (1H, m), 6.05 (1H, m), 6.14 (1H, d, J=9Hz), 8.31 (1H, d, J=9Hz)

Preparation 20

To a solution of 5-allyloxy-2-butyl-3-[(2′-cyanobiphenyl-4-yl)methyl]-3H-imidazo[4,5-b]pyridine (600 mg) in formic acid (1 ml) was added tetrakis(triphenylphosphine)palladium(0) (100 mg) in one portion. The suspension was stirred at 90 °C for 14 hours and evaporated under reduced pressure. The residue was dissolved in ethyl acetate. The mixture was washed with aqueous saturated sodium bicarbonate solution, dried, and concentrated to give a residue, which was purified by silica gel chromatography eluted with chloroform to give 2-butyl-3-[(2′-cyanobiphenyl-4-yl)methyl]-5-hydroxy-3H-imidazo[4,5-b]pyridine (280 mg) as crystals.

mp : 195-196 °C

NMR ($CDCl_3$, δ) : 0.89 (3H, t, J=7.5Hz), 1.41 (2H, m), 1.80 (2H, m), 2.79 (2H, t, J=7.5Hz), 3.48 (1H, s), 5.45 (2H, s), 6.72 (1H, d, J=9Hz), 7.20 (2H, d, J=8Hz), 7.40-7.55 (4H, m), 7.62 (1H, m), 7.75 (1H, dd, J=1, 8Hz), 7.97 (1H, d, J=9Hz)

Preparation 21

A mixture of 2′-cyano-4-ethylbiphenyl (4.6 g), N-bromosuccinimide (4.35 g), and 2,2′-azobisisobutyronitrile (50 mg) in tetrachloromethane (100 ml) was refluxed for 3 hours. The cooled mixture was washed with water, dried, and concentrated in vacuo. The residue was purified by chromatography on silica gel (elution by chloroform) to give 4′-(1-bromoethyl)-2-cyanobiphenyl (4.6 g).

NMR ($CDCl_3$, δ) : 2.10 (3H, d, J=7Hz), 5.29 (1H, q, J=7Hz), 7.45-7.95 (8H, m)

Preparation 22

The following compound was obtained according to a similar manner to that of Preparation 6.

2-Cyano-3′-fluoro-4′-methylbiphenyl

mp : 82-84 °C

NMR ($CDCl_3$, δ) : 2.38 (3H, d, J=2Hz), 7.2-7.9 (7H, m)

Preparation 23

The following compound was obtained according to a similar manner to that of Preparation 21.

4-Bromomethyl-2-cyano-3-fluorobiphenyl

mp : 99-102 °C

NMR ($CDCl_3$, δ) : 4.58 (2H, d, J=1Hz), 7.2-8.0 (7H, m)

Preparation 24

The following compounds were obtained according to a similar manner to that of Preparation 1 or 2.

(1) 2-Butyl-3-[(2′-cyano-3-fluorobiphenyl-4-yl)methyl]- 3H-imidazo[4,5-b]pyridine

NMR ($CDCl_3$, δ) : 0.95 (3H, t, J=7Hz), 1.3-1.6 (2H, m), 1.75-0.95 (2H, m), 2.90 (2H, t, J=7Hz), 5.62 (2H, s), 7.01 (1H, t, J=8Hz), 7.2-7.5 (5H, m), 7.63 (1H, m), 7.76 (1H, m), 8.03 (1H, dd, J=1,8Hz), 8.37 (1H, dd, J=1,8Hz)

(2) 2-Butyl-3-[(2′-cyano-3-fluorobiphenyl-4-yl)methyl]-7-methyl-3H-imidazo[4,5-b]pyridine

NMR ($CDCl_3$, δ) : 0.93 (3H, t, J=7Hz), 1.45 (2H, m), 1.78 (2H, m), 2.73 (3H, s), 2.95 (2H, t, J=7Hz), 5.62 (2H, s), 6.95-7.8 (8H, m), 8.27 (1H, d, J=5Hz)

Example 1

A mixture of 2-butyl-3-[(2′-cyanobiphenyl-4-yl)methyl]-3H-imidazo[4,5-b]pyridine (500 mg) and trimethyltin azide (841 mg) in xylene (10 ml) was heated at 120° C for 3 days and cooled to room temperature. To the solution were added 4N-hydrogen chloride in 1,4-dioxane (4 ml) and water (15 ml) and the mixture was stirred for 30 minutes. The solution was washed with ether and the aqueous phase was evaporated in vacuo. The residue was dissolved in a mixture of water and methanol and the solution was adjusted to pH 8 with 1N-aqueous sodium hydroxide. The solvent was evaporated in vacuo and the residue was triturated with methanol. The insoluble material was filtered off and the filtrate was evaporated in vacuo. The residue was chromatographed on a silica gel column with a mixture of chloroform and methanol (10:1) as an eluent. The object fractions were collected and evaporated in vacuo. The residue was solidified with water to give 2-butyl-3-[[2′-(1H-tetrazol-5-yl)biphenyl-4-yl]methyl]-3H-imidazo[4,5-b]pyridine (350 mg) as a white powder.

mp : 133-135° C

NMR (DMSO-$d_6$, δ) : 0.72 (3H, t, J=7.5Hz), 1.31 (2H, m), 1.65 (2H, m), 2.57 (2H, t, J=7.5Hz), 5.36 (2H, s), 6.85 (2H, d, J=8.5Hz), 6.96 (2H, d, J=8.5Hz), 7.13 (1H, dd, J=5, 8.5Hz), 7.35 (1H, m), 7.50-7.65 (2H, m), 7.93 (1H, m), 8.27 (1H, dd, J=5, 1Hz)

Example 2

The following compound was obtained according to a similar manner to that of Example 1.

8-Butyl-9-[[2′-(1H-tetrazol-5-yl)biphenyl-4-yl]methyl]purine

mp : 115-120° C

NMR (DMSO-$d_6$, δ) : 0.83 (3H, t, J=7.5Hz), 1.35 (2H, m), 1.70 (2H, m), 2.35 (2H, t, J=7.5Hz), 5.40 (2H, s), 6.84 (2H, d, J=8.5Hz), 6.98 (2H, d, J=8.5Hz), 7.37 (1H, m), 7.50-7.65 (2H, m), 7.96 (1H, m), 8.47 (1H, s), 8.55 (1H, s)

Example 3

The following compounds were obtained according to a similar manner to that of Example 1.

(1) 2-Pentyl-3-[[2′-(1H-tetrazol-5-yl)biphenyl-4-yl]methyl]-3H-imidazo[4,5-b]pyridine

mp : 119-121° C

NMR (DMSO-$d_6$, δ) : 0.84 (3H, t, J=7.5Hz), 1.20-1.4 (4H, m), 1.60-1.70 (2H, m), 2.82 (1H, t, J=7.5Hz), 5.52 (2H, s), 7.05 (2H, d, J=8.5Hz), 7.11 (2H, d, J=8.5Hz), 7.27 (1H, dd, J=7.5Hz and 5Hz), 7.45-7.70 (4H, m), 8.02 (1H, dd, J=7.5Hz and 1Hz), 8.31 (1H, dd, J=5Hz and 1Hz)

(2) 2-Propyl-3-[[2′-(1H-tetrazol-5-yl)biphenyl-4-yl]methyl]-3H-imidazo[4,5-b]pyridine

mp : 126-130° C

NMR (DMSO-$d_6$, δ) : 0.93 (3H, t, J=7.5Hz), 1.72 (2H m), 2.81 (2H, t, J=7.5Hz), 5.52 (2H, s), 7.05 (2H, d, J=8.5Hz), 7.14 (2H, d, J=8.5Hz), 7.17 (1H, dd, J=7.5Hz and 5Hz), 7.55-7.70 (4H, m), 8.02 (1H, d, J=7.5Hz), 8.32 (1H, d, J=5Hz)

(3) 2-Butyl-1-[[2′-(1H-tetrazol-5-yl)biphenyl-4-yl]methyl]-1H-naphth[2,3-d]imidazole

mp : 215.5-217° C

NMR (DMSO-$d_6$, δ) : 0.89 (3H, t, J=6.5Hz), 1.30-1.52 (2H, m), 1.77-1.86 (2H, m), 2.89 (2H, t, J=7.5Hz), 5.59 (2H, s), 7.08 (4H, s), 7.31-7.71 (6H, m), 7.88-8.04 (3H, m), 8.12 (1H, s)

(4) 2-Butyl-1-[[2′-(1H-tetrazol-5-yl)biphenyl-4-yl]methyl]-1H-naphth[1,2-d]imidazole

mp : 142-158° C

NMR (DMSO-$d_6$, δ) : 0:89 (3H, t, J=7.0Hz), 1.29-1.51 (2H, m), 1.63-1.84 (2H, m), 2.90 (2H, t, J=7.5Hz), 5.62 (2H, s), 7.03 (4H, s), 7.37-7.78 (8H, m), 7.97 (1H, d, J=9.0Hz), 8.44 (1H, d, J=9.0Hz)

Example 4

To a solution of 2-butyl-3-[[2′-(1H-tetrazol-5-yl)biphenyl-4-yl]methyl]-3H-imidazo[4,5b]pyridine (28 g) in hot ethanol (300 ml) was added a solution of sodium bicarbonate (5.7 g) in water (140 ml) in one portion. The mixture was stirred for half an hour at ambient temperature and concentrated in vacuo. The residue was dissolved in ethanol (400 ml). The ethanolic solution was filtered and concentrated in vacuo. The

residue was dissolved in hot acetonitrile (400 ml). The precipitates were filtered and washed with acetonitrile to give sodium salt of 2-butyl-3-[[2′-(1H-tetrazol-5-yl)biphenyl-4-yl]methyl]-3H-imidazo[4,5-b]pyridine (trihydrate) (25.5 g) as white crystals.
mp : 100-101 °C
NMR ($D_2O$, δ) : 0.63 (3H, t, J=7.5Hz), 1.03 (2H, m), 1.34 (2H, m), 2.48 (2H, t, J=7.5Hz), 5.16 (2H, s), 6.68 (4H, s), 6.96 (1H, dd, J=1.0, 7Hz), 7.11 (1H, dd, J=5, 8Hz), 7.24 (1H, dt, J=1.0, 7.0Hz), 7.35 (1H, dt, J=1.0, 7.0Hz), 7.52 (1H, dd, J=1.0, 7.0Hz), 7.85 (1H, dd, J=1.0, 8.0Hz), 8.07 (1H, dd, J=1.0, 5.0Hz)

Example 5

6-Bromo-2-butyl-3-[(2′-cyanobiphenyl-4-yl)methyl]3H-imidazo[4,5-b]pyridine (500 mg), sodium azide (219 mg), triethylamine hydrochloride (231 mg) and 1-methyl-2-pyrrolidone (2.5 ml) were combined under nitrogen atmosphere. The reaction mixture was stirred at 120 °C for 20 hours. After being cooled to ambient temperature, sodium azide (220 mg) and triethylamine hydrochloride (230 mg) were added to the solution, and then the reaction mixture was stirred at 120 °C for 69 hours. After being cooled to ambient temperature, ethyl acetate and water, and then 1N-hydrochloric acid were added to the solution until pH 4.5. The organic layer was separated, and the aqueous layer was extracted with ethyl acetate, and the organic layer was washed successively with 5% aqueous sodium nitrite (twice), water and brine and dried over magnesium sulfate. The solvent was evaporated in vacuo and the residue was chromatographed on a silica gel column with a mixture of methanol and chloroform (1:20 → 1:10) as an eluent to give 6-brown-2- butyl-3-[[2′-(1H-tetrazol-5-yl)biphenyl-4-yl]methyl]-3H-imidazo[4,5-b]pyridine (49 mg).
mp : 195-197. 5 °C
NMR (DMSO-$d_6$, δ) : 0.87 (3H, t, J=7.5Hz), 1.22-1.47 (2H, m), 1.59-1.78 (2H, m), 2.83 (2H, t, J=7.5Hz), 5.52 (2H, s), 7.05 (2H, d, J=9.0Hz), 7.12 (2H, d, J=9.0Hz), 7.47-7.73 (4H, m), 8.31 (1H, d, J=1.5Hz), 8.41 (1H, d, J=1.5Hz)

Example 6

The following compounds were obtained according to a similar manner to that of Example 1 or 5.

(1) 2-Butyl-7-methyl-3-[[2′-(1H-tetrazol-5-yl)biphenyl-4-yl]methyl]-3H-imidazo[4,5-b]pyridine
mp : 100-103 °C
NMR (DMSO-$d_6$, δ) : 0.82 (3H, t, J=7.5Hz), 1.32 (2H, m), 1.64 (2H, m), 2.54 (3H, s), 2.79 (2H, t, J=7.5Hz), 5.48 (2H, s), 7.00-7.10 (5H, m), 7.50-7.70 (4H, m), 8.15 (1H, d, J=5Hz)

(2) 2-Butyl-1-[[2′-(1H-tetrazol-5-yl)biphenyl-4-yl]methyl]-1H-imidazo[4,5-6]pyrazine
mp : 110-115 °C
NMR (DMSO-$d_6$, δ) : 0.85 (3H, t, J=7.5Hz), 1.38 (2H, m), 1.71 (2H, m), 2.90 (2H, t, J=7.5Hz), 5.54 (2H, s), 7.06 (2H, d, J=8Hz), 7.16 (2H, d, J=8Hz), 7.50-7.70 (4H, m), 8.35 (1H, d, J=2Hz), 8.47 (1H, d, J=2Hz)

(3) 2-Propylthio-3-[[2′-(1H-tetrazol-5-yl)biphenyl-4-yl]methyl]-3H-imidazo[4,5-b]pyridine
mp : 75-80 °C
NMR (DMSO-$d_6$, δ) : 0.98 (3H, t, J=7.5Hz), 1.76 (2H, m), 3.33 (2H, t, J=7.5Hz), 5.38 (2H, s), 7.07 (2H, d, J=9Hz), 7.20-7.35 (3H, m), 7.55-7.80 (4H, m), 8.00 (1H, dd, J=1, 9Hz), 8.27 (1H, dd, J=1, 5Hz)

(4) 2-Ethylthio-3-[[2′-(1H-tetrazol-5-yl)biphenyl-4-yl]methyl]-3H-imidazo[4,5-b]pyridine
mp : 103-106 °C
NMR (DMSO-$d_6$, δ) : 1.19 (3H, t, J=7.5Hz), 3.18 (2H, q, J=7.5Hz), 5.18 (2H, s), 6.83 (2H, d, J=8Hz), 6.99 (2H, d, J=8Hz), 7.06 (1H, dd, J=5, 9Hz), 7.30-7.50 (4H, m), 7.79 (1H, dd, J=1, 8Hz), 8.07 (1H, dd, J=1, 5Hz)

(5) 2-Butyl-5-chloro-3-[[2′-(1H-tetrazol-5-yl)biphenyl-4-yl]methyl]-3H-imidazo[4,5-b]pyridine
mp : 194-196 °C
NMR (DMSO-$d_6$, δ) : 0.84 (3H, t, J=7.5Hz), 1.29 (2H, m), 1.63 (2H, m), 2.57 (2H, t, J=7.5Hz), 5.38 (2H, s), 6.94 (2H, d, J=8Hz), 7.02 (2H, d, J=8Hz), 7.14 (1H, d, J=9Hz), 7.39 (1H, m), 7.52 (1H, d, J=9Hz), 7.58 (2H, m), 7.97 (1H, m)

(6) 2-Butyl-5-methoxy-3-[[2′-(1H-tetrazol-5-yl)biphenyl-4-yl]methyl]-3H-imidazo[4,5-b]pyridine
mp : 248-249 °C (dec.)
NMR (DMSO-$d_6$, δ) : 0.87 (3H, t, J=7.5Hz), 1.33 (2H, m), 1.58 (2H, m), 2.78 (2H, t, J=7.5Hz), 3.89 (3H, s), 5.42 (2H, 2), 6.68 (1H, d, J=9Hz), 7.08 (2H, d, J=8Hz), 7.20 (2H, d, J=8Hz), 7.50-7.70 (4H, m), 7.90

(1H, d, J=9Hz)

(7) 2-Butyl-3-[1-[2′-(1H-tetrazol-5-yl)biphenyl-4-yl]ethyl]-3H-imidazo[4,5-b]pyridine

mp : 183-186° C

NMR (DMSO-$d_6$, δ) : 0.83 (3H, t, J=7.5Hz), 1.39 (2H, m), 1.65 (2H, m), 2.04 (3H, d, J=7Hz), 2.79 (2H, t, J=7.5Hz), 6.05 (1H, q, J=7Hz), 7.04 (2H, d, J=8Hz), 7.20-7.30 (3H, m), 7.50-7.75 (4H, m), 7.97 (1H, dd, J=1, 8Hz), 8.26 (1H, dd, J=1, 5Hz)

(8) 2-Butyl-3-[[2′-(1H-tetrazol-5-yl)biphenyl-4-yl]methyl]-5-(2,2,2-trifluoroethoxy)-3H-imidazo[4,5-b]pyridine

mp : 249-250° C

NMR (DMSO-$d_6$, δ) : 0.87 (3H, t, J=7, 5Hz), 1.35 (2H, m), 1.66 (2H, m), 2.79 (2H, t, J=7.5Hz), 5.04 (2H, q, J=9, 5Hz), 5.46 (2H, s), 6.81 (1H, d, J=9Hz), 7.07 (2H, d, J=8Hz), 7.20 (2H, d, J=8Hz), 7.50-7.75 (4H, m), 8.01 (1H, d, J=9Hz)

(9) 2-Butyl-5-hydroxy-3-[[2′-(1H-tetrazol-5-yl)biphenyl-4-4-yl]methyl]-3H-imidazo[4,5-b]pyridine

mp : 256-258° C

NMR (DMSO-$d_6$, δ) : 0.83 (3H, t, J=7.5Hz), 1.31 (2H, m), 1.62 (2H, m), 2.71 (2H, t, J=7.5Hz), 5.38 (2H, s), 6.52 (1H, d, J=9Hz), 7.04 (4H, s), 7.50-7.70 (4H, m), 7.84 (1H, d, J=9Hz)

(10) 3-[[2′-(1H-tetrazol-5-yl)biphenyl-4-yl]methyl]-2-[2-(trifluoromethyl)propyl]-3H-imidazo[4,5-b]pyridine

mp : 96-101 ° C

NMR (DMSO-$d_6$, δ) : 1.05 (3H, d, J=7Hz), 2.90 (1H, dd, J=15, 9.5H:), 3.10-3.30 (2H, m), 5.52 (1H, d, J=15Hz), 5.63 (1H, d, J=15Hz), 7.05 (2H, d, J=8Hz), 7.12 (2H, d, J=8Hz), 7.30 (1H, dd, J=9, 5Hz), 7.50-7.70 (4H, m), 8.07 (1H, dd, J=8, 1Hz), 8.36 (1H, dd, J=5, 1Hz)

(11) 2-Cyclopropyl-3-[[2′-(1H-tetrazol-5-yl)biphenyl-4- yl]methyl]-3H-imidazo[4,5-b]pyridine

mp : 95-98° C

NMR (DMSO-$d_6$, δ) : 1.03 (4H, d, J=6.0Hz), 2.27 (1H, quint, J=6.0Hz), 5.62 (2H, s), 7.04 (2H, d, J=9.0Hz), 7.20 (2H, d, J=9.0Hz), 7.13-7.28 (1H, m), 7.47-7.73 (4H, m), 7.91 (1H, dd, J=8.0, 1.0Hz), 8.27 (1H, dd, J=5.0, 1.0Hz)

(12) 2-Butyl-1-[[2′-(1H-tetrazol-5-yl)biphenyl-4-yl]methyl]-1H-imidazo[4,5-b]pyridine

mp : 137-143.5° C

NMR (DMSO-$d_6$, δ) : 0.88 (3H, t, J=7.5Hz), 1.25-1.47 (2H, m), 1.61-1.80 (2H, m), 2.86 (2H, t, J=7.5Hz), 5.53 (2H, s), 7.06 (4H, s), 7.20 (1H, dd, J=9.0, 5.0Hz), 7.45-7.72 (4H, m), 7.92 (1H, dd, J=9.0, 1.0Hz), 8.35 (1H, dd, J=5.0, 1.0Hz)

(13) 2-Cyclohehyl-3-[[2′-(1H-tetrazol-5-yl)biphenyl-4-yl]methyl]-3H-imidazo[4,5-b]pyridine

mp : 193-195° C

NMR (DMSO-$d_6$, δ) : 1.09-1.85 (10H, m), 2.84-3.06 (1H, m), 5.55 (2H, s), 7.03 (2H, d, J=9.0Hz), 7.13 (2H, d, J=9.0Hz), 7.28 (1H, dd, J=8.0, 5.0Hz), 7.45-7.73 (4H, m), 8.00 (1H, d, J=8.0Hz), 8.31 (1H, d, J=5.0Hz)

(14) 2-Butyl-6-methyl-3-[[2′-(1H-tetrazol-5-yl)biphenyl-4-yl]methyl]-3H-imidazo[4,5-b]pyridine

mp : 158-160° C

NMR (DMSO-$d_6$, δ) : 0.69 (3H, t, J=7.5Hz), 1.07-1.28 (2H, m), 1.41-1.60 (2H, m), 2.25 (3H, s), 2.62 (2H, t, J=7.5Hz), 5.32 (2H, s), 6.88 (2H, d, J=9.0Hz), 6.94 (2H, d, J=9.0Hz), 7.30-7.57 (4H, m), 7.65 (1H, d, J=1.0Hz), 8.00 (1H, d, J=1.0Hz)

(15) 6-Bromo-2-butyl-7-methyl-3-[[2′-(1H-tetrazol-5-yl)biphenyl-4-yl]methyl]-3H-imidazo[4,5-b]pyridine

mp : 204. 5-207° C

NMR (DMSO-$d_6$, δ) : 0.85 (3H, t, J=7.5Hz), 1.23-1.43 (2H, m), 1.56-1.73 (2H, m), 2.60 (3H, s), 2.82 (2H, t, J=7.5Hz), 5.51 (2H, s), 7.05 (2H, d, J=9.0Hz), 7.11 (2H, d, J=9.0Hz), 7.45-7.72 (4H, m), 8.39 (1H, s)

Example 7

The following compound was obtained according to a similar manner to that of Example 4.

Sodium salt of 2-butyl-7-methyl-3-[[2′-(1H-tetrazol-5-yl)biphenyl-4-yl]methyl]-3H-imidazo[4,5-b]pyridine

mp : 110-114° C

NMR ($D_2O$, δ) : 0.58 (3H, t, J=5.5Hz), 1.01 (2H, m), 1.26 (2H, m), 2.35 (3H, s), 2.49 (2H, t, J=7.5Hz), 5.10 (2H, s), 6.61 (4H, s), 6.78 (1H, d, J=5Hz), 6.89 (1H, d, J=8Hz), 7.20 (1H, t, J=8Hz), 7.31 (1H, t, J=8Hz), 7.48 (1H, d, J=8Hz), 7.81 (1H, d, J=5Hz)

Example 8

2-Butyl-5-chloro-3-[[2′-(1H-tetrazol-5-yl)biphenyl-4-yl]methyl]-3H-imidazo[4,5-b]pyridine was obtained from 6-chloro-3-nitro-2-[N-[[2′-(1-trityl-1H-tetrazol-5-yl)biphenyl-4-yl]methyl]-N-valerylamino]pyridine according to a similar manner to that of Preparation 9.

NMR ($CDCl_3$, δ) : 0.84 (3H, t, J=7.5Hz), 1.29 (2H, m), 1.63 (2H, m), 2.57 (2H, t, J=7.5Hz), 5.38 (2H, s), 6.94 (2H, d, J=8Hz), 7.02 (2H, d, J=8Hz), 7.14 (1H, d, J=9Hz), 7.39 (1H, m), 7.52 (1H, d, J=9Hz), 7.58 (2H, m), 7.97 (1H, m)

Example 9

The following compounds were obtained according to a similar manner to that of Example 1.

(1) 2-Butyl-3-[[3-fluoro-2′-(1H-tetrazol-5-yl)biphenyl-4-yl]methyl]-3H-imidazo[4,5-b]pyridine

NMR (DMSO-$d_6$, δ) : 0.87 (3H, t, J=7Hz), 1.25-1.45 (2H, m), 1.6-1.8 (2H, m), 2.83 (2H, t, J=7Hz), 5.55 (2H, s), 6.75-6.9 (2H, m), 7.02 (1H, dd, J=11Hz), 7.25 (1H, dd, J=5Hz and 8Hz), 7.45-7.75 (4H, m), 8.01 (1H, dd, J=1Hz and 8Hz), 8.28 (1H, dd, J=1Hz and 5Hz)

(2) 2-Butyl-3-[[3-fluoro-2′-(1H-tetrazol-5-yl)biphenyl-4-yl]methyl]-7-methyl-3H-imidazo[4,5-b]pyridine

NMR (DMSO-$d_6$, δ) : 0.87 (3H, t, J=7Hz), 1.35 (2H, m), 1.68 (2H, m), 2.54 (3H, s), 2.82 (2H, t, J=7Hz), 5.52 (2H, s), 6.7-7.7 (8H, m), 8.13 (1H, d, J=5Hz)

Example 10

The following compound was obtained according to a similar manner to that of Example 4.

Sodium salt of 2-butyl-3-[[3-fluoro-2′-(1H-tetrazol-5-yl)biphenyl-4-yl]methyl]-3H-imidazo[4,5-b]pyridine

NMR ($CD_3OD$, δ) : 0.82 (3H, t, J=7Hz), 1.30 (2H, m), 1.57 (2H, m), 2.53 (3H, s), 2.78 (2H, t, J=7Hz), 5.47 (2H, s), 6.6-8.1 (9H, m)

Example 11

2-Butyl-3-[[2′-(1H-tetrazol-5-yl)biphenyl-4-yl]methyl]-3H-imidazo[4,5-b]pyridine was obtained from 2-butyl-3H-imidazo[4,5-b]pyridine and 4′-bromomethyl-2-(1H-tetrazol-5-yl)biphenyl in a conventional manner.

NMR (DMSO-$d_6$, δ): 0.72 (3H, t, J=7.5Hz), 1.31 (2H, m), 1.65 (2H, m), 2.57 (2H, t, J=7.5Hz), 5.36 (2H, s), 6.85 (2H, d, J=8.5Hz), 6.96 (2H, d, J=8.5Hz), 7.13 (1H, dd, J=5, 8.5Hz), 7.35 (1H, m), 7.50-7.65 (2H, m), 7.93 (1H, m), 8.27 (1H, dd, J=5, 1Hz)

Example 12

2-Butyl-3-[[2′-(1H-tetrazol-5-yl)biphenyl-4-yl]methyl]-3H-imidazo[4,5-b]pyridine was obtained from 2-butyl-3-[[2′-(1-trityl-1H-tetrazol-5-yl)biphenyl-4-yl]methyl]-3H-imidazo[4,5-b]pyridine in a conventional manner.

NMR (DMSO-$d_6$, δ): 0.72 (3H, t, J=7.5Hz), 1.31 (2H, m), 1.65 (2H, m), 2.57 (2H, t, J=7.5Hz), 5.36 (2H, s), 6.85 (2H, d, J=8.5Hz), 6.96 (2H, d, J=8.5Hz), 7.13 (1H, dd, J=5, 8.5Hz), 7.35 (1H, m), 7.50-7.65 (2H, m), 7.93 (1H, m), 8.27 (1H, dd, J=5, 1Hz)

Example 13

2-Butyl-3-[[2′-(1H-tetrazol-5-yl)biphenyl-4-yl]methyl]-3H-imidazo[4,5-b]pyridine was obtained from 3-nitro-2-[N-[[2′-(1-trityl-1H-tetrazol-5-yl)biphenyl-4-yl]methyl]-N-valerylamino]pyridine according to a similar manner to that of Preparation 9.

NMR (DMSO-$d_6$, δ): 0.72 (3H, t, J=7.5Hz), 1.31 (2H, m), 1.65 (2H, m), 2.57 (2H, t, J=7.5Hz), 5.36 (2H, s), 6.85 (2H, d, J=8.5Hz), 6.96 (2H, d, J=8.5Hz), 7.13 (1H, dd, J=5, 8.5Hz), 7.35 (1H, m), 7.50-7.65 (2H, m), 7.93 (1H, m), 8.27 (1H, dd, J=5, 1Hz)

**Claims**

1. A compound of the formula :

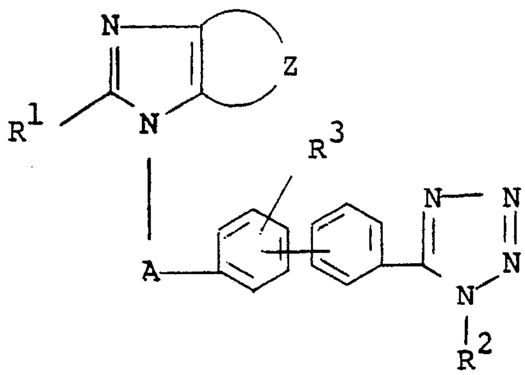

wherein $R^1$ is lower alkyl, halo(lower)alkyl, cyclo(lower)alkyl or lower alkylthio,
$R^2$ is hydrogen or imino-protective group,
$R^3$ is hydrogen or halogen,
A is lower alkylene, and

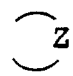

is a group of the formula :

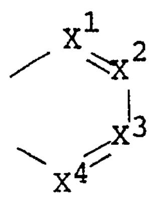 , 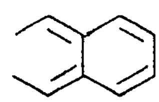 or 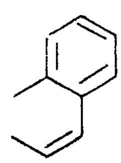

in which
$X^1$, $X^2$, $X^3$ and $X^4$ are each
N or $CR^4$, in which $R^4$ is hydrogen, lower alkyl, lower alkoxy, halogen, halo(lower)alkyl, esterified carboxy, halo(lower)alkoxy or hydroxy,
provided that at least one of $X^1$, $X^2$, $X^3$ and $X^4$ is N,
and pharmaceutically acceptable salts thereof.

2. A compound of claim 1, wherein
$R^2$ is hydrogen or ar(lower)alkyl, and

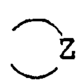

is a group of the formula

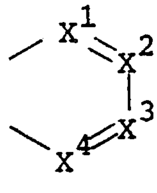

in which $X^1$, $X^2$, $X^3$ and $X^4$ are each N or $CR^4$,
in which $R^4$ is hydrogen, lower alkyl, lower alkoxy, halogen, halo(lower)alkoxy or hydroxy.

3. A compound of claim 2, wherein
$X^1$, $X^2$ and $X^3$ are each $CR^4$, in which
$R^4$ is as defined in claim 2, and
$X^4$ is N.

4. A compound of claim 3, wherein
$R^1$ is propyl, butyl, pentyl, trifluoromethylpropyl, cyclopropyl, cyclohehyl, ethylthio or propylthio,
$R^2$ is hydrogen,
$R^3$ is hydrogen or fluoro,
A is methylene or methylmethylene, and
$R^4$ is hydrogen, methyl, methoxy, chloro, bromo, trifluoroethoxy or hydroxy.

5. A compound of claim 4, wherein
$R^1$ is butyl,
$R^3$ is hydrogen,
A is methylene,
$X^1$ is $CR^4$, in which $R^4$ is hydrogen or methyl, and $X^2$ and $X^3$ are each CH.

6. A compound of claim 5, which is
2-butyl-3-[[2'-(1H-tetrazol-5-yl)biphenyl-4-yl]methyl]-3H-imidazo[4,5-b]pyridine or its sodium salt.

7. A compound of claim 5, which is
2-butyl-7-methyl-3-[[2'-(1H-tetrazol-5-yl)biphenyl-4-yl]methyl]-3H-imidazo[4,5-b]pyridine or its sodium salt.

8. A compound of claim 1, wherein

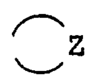

is a group of the formula

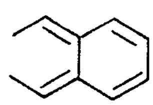

or

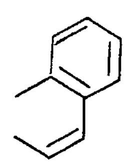

.

9. A process for preparing a compound of the formula

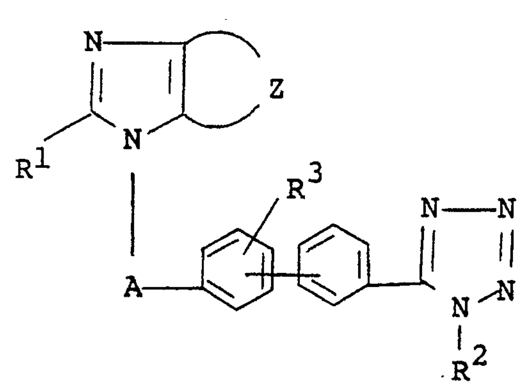

wherein $R^1$ is lower alkyl, halo(lower)alkyl, cyclo(lower)alkyl or lower alkylthio,
$R^2$ is hydrogen or imino-protective group,
$R^3$ is hydrogen or halogen,
A is lower alkylene, and

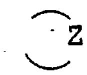

is a group of the formula :

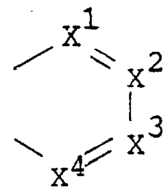 , 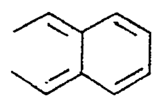 or 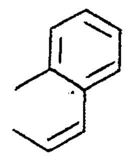

in which
$X^1$, $X^2$, $X^3$ and $X^4$ are each
N or $CR^4$, in which $R^4$ is hydrogen, lower alkyl, lower alkoxy, halogen, halo(lower)alkyl, esterified carboxy, halo(lower)alkoxy or hydroxy,
provided that at least one of $X^1$, $X^2$, $X^3$ and $X^4$ is N,
or a salt thereof which comprises

(1) subjecting a compound of the formula

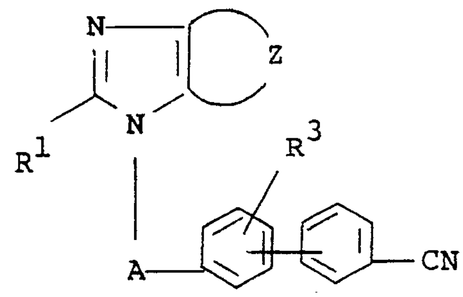

wherein $R^1$, $R^3$, A and

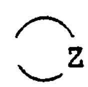

are each as defined above,
to formation reaction of a tetrazole group,
to give a compound of the formula :

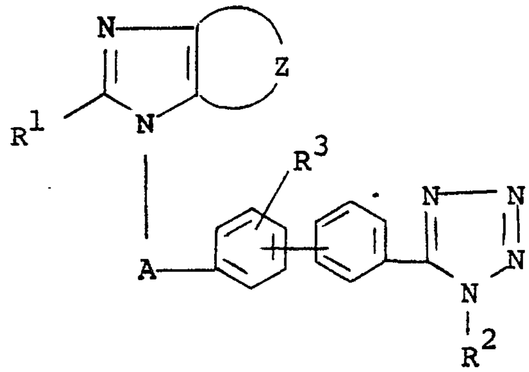

wherein $R^1$, $R^2$, $R^3$, A and

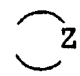

are each as defined above,
or a salt thereof, or
(2) reacting a compound of the formula :

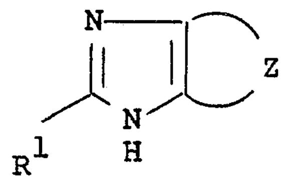

wherein $R^1$ and

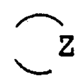

are each as defined above,
or a salt thereof, with a compound of the formula :

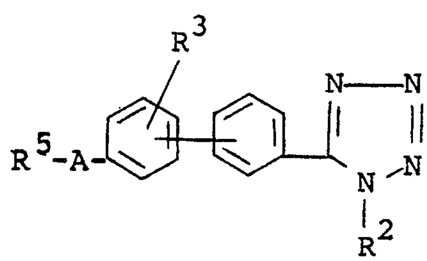

wherein $R^2$, $R^3$ and A are each as defined above, and $R^5$ is acid residue,
or a salt thereof, to give a compound of the formula:

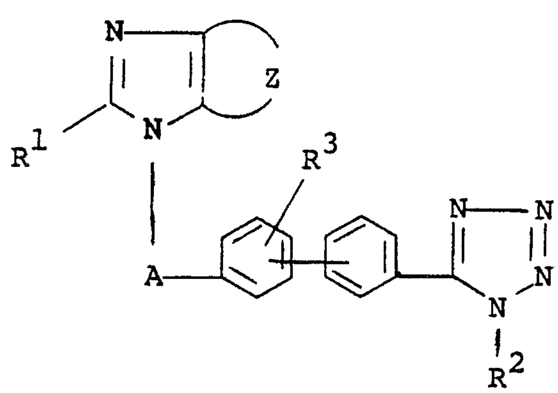

wherein $R^1$, $R^2$, $R^3$, A and

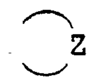

are each as defined above,
or a salt thereof, or
(3) subjecting a compound of the formula

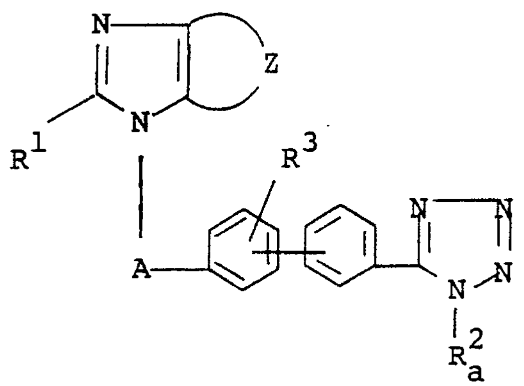

wherein $R^1$, $R^3$, A and

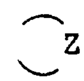

are each as defined above,
and
$R_a^2$ is imino-protective group,
to removal reaction of the imino-protective group,
to give a compound of the formula :

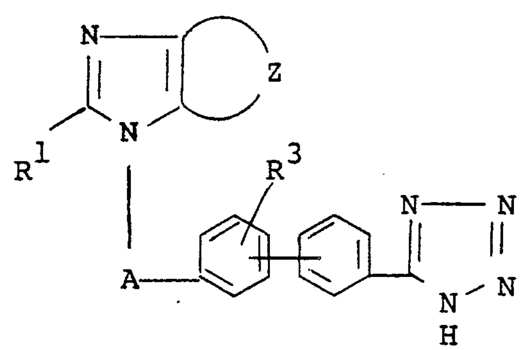

wherein $R^1$, $R^3$, A and

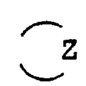

are each as defined above,
or a salt thereof, or
(4) subjecting a compound of the formula :

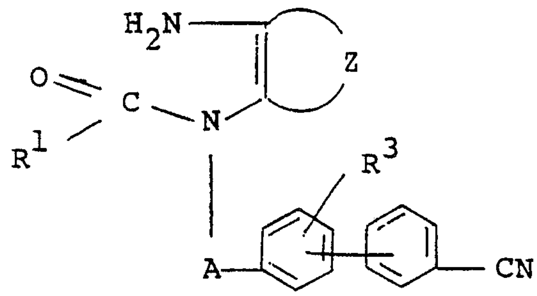

wherein $R^1$, $R^3$, A and

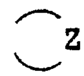

are each as defined above, or a salt thereof, to formation reaction of a imidazole group and a tetrazole group, to give a compound of the formula :

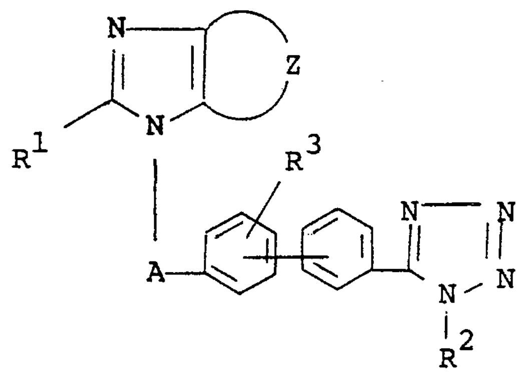

wherein $R^1$, $R^2$, $R^3$ A and

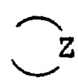

are each as defined above, or a salt thereof, or (5) subjecting a compound of the formula :

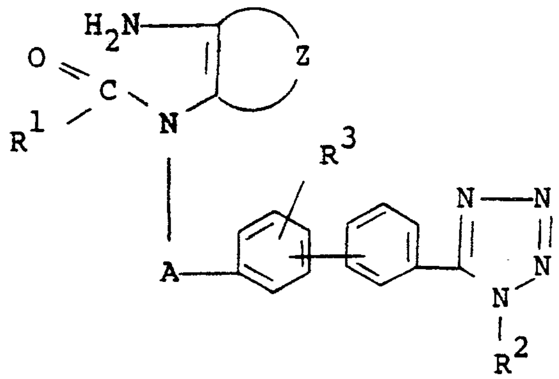

wherein $R^1$, $R^2$, $R^3$, A and

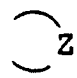

are each as defined above,
or a salt thereof, to formation reaction of a imidazole group, to give a compound of the formula

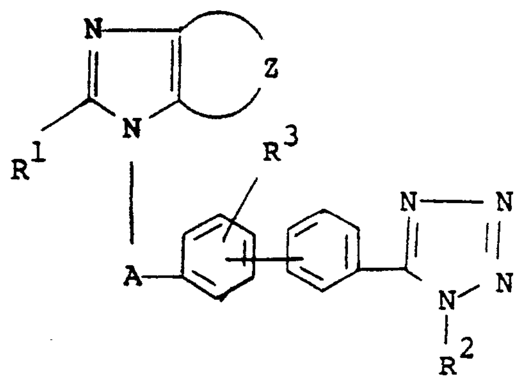

wherein $R^1$, $R^2$, $R^3$, A and

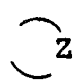

are each as defined above,
or a salt thereof.

10. A pharmaceutical composition comprising a compound of claim 1 or pharmaceutically acceptable salt thereof in association with a pharmaceutically acceptable, substantially non-toxic carrier or excipient.

11. Use of a compound of claim 1 for pharmaceutically acceptable salt thereof for the manufacture of a medicament for therapeutically treating or preventing hypertension or heart failure.

12. Use of a compound of claim 1 or pharmaceutically acceptable salts thereof in treating or preventing hypertension or heart failure.

13. A compound of claim 1 or pharmaceutically acceptable salt thereof for use as a medicament.

14. A compound of claim 1 or pharmaceutically acceptable salt thereof for use as an angiotensin II antagonist.

15. Use of a compound of claim 1 for manufacturing a medicament for treating or preventing angiotensin II mediated diseases.

16. A process for preparing a pharmaceutical composition which comprises admixing a compound of claim

1 with a pharmaceutically acceptable substantially non-toxic carrier or excipient.

European Patent Office

# EUROPEAN SEARCH REPORT

Application Number

EP 90 12 0543

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 291 969 (E.I. DU PONT DE NEMOURS AND CO.)<br>--- | | C 07 D 471/04<br>A 61 K 31/44<br>C 07 D 487/04<br>C 07 D 473/00<br>C 07 D 473/40<br>C 07 D 403/10<br>A 61 K 31/52<br>A 61 K 31/505<br>A 61 K 31/415<br>A 61 K 31/495<br>A 61 K 31/50 |
| A | EP-A-0 253 310 (E.I. DU PONT DE NEMOURS AND CO.)<br>--- | | |
| E | EP-A-0 400 835 (MERCK & CO., INC.)<br>* Pages 2-11, line 16; page 11, line 26; page 32; figures example 9; pages 12-25 *<br>----- | 1,8-16 | |
| | | | TECHNICAL FIELDS SEARCHED (Int. Cl.5) |
| | | | C 07 D 471/00<br>A 61 K 31/00<br>C 07 D 487/00<br>C 07 D 473/00<br>C 07 D 403/00 |

The present search report has been drawn up for all claims

| Place of search | Date of completion of the search | Examiner |
|---|---|---|
| THE HAGUE | 31-01-1991 | DE BUYSER I.A.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)